# EUROPÄISCHE PATENTSCHRIFT

(11) **EP 1 196 605 B1**
(45) Veröffentlichungstag und Bekanntmachung des Hinweises auf die Patenterteilung: **24.09.2008**
(21) Anmeldenummer: 00956319.8
(22) Anmeldetag: 27.07.2000
(51) Int. Cl.: C12N 15/53, C12N 9/02, C12N 1/21, C12P 17/10, C12P 17/16, C12P 7/22

(54) **NEUE CYTOCHROM P450-MONOOXYGENASEN UND DEREN VERWENDUNG ZUR OXIDATION VON ORGANISCHEN VERBINDUNGEN**
NOVEL CYTOCHROME P450 MONOOXYGENASES AND THEIR USE FOR OXIDIZING ORGANIC COMPOUNDS
MONOOXYGENASES A CYTOCHROME P450 ET LEUR UTILISATION POUR L'OXYDATION DE COMPOSES ORGANIQUES

(30) Priorität: 27.07.1999 DE 19935115; 18.11.1999 DE 19955605; 22.03.2000 DE 10014085
(43) Veröffentlichungstag der Anmeldung: 17.04.2002
(73) Patentinhaber: BASF SE, 67056 Ludwigshafen (DE)
(72) Erfinder: HAUER, Bernhard, D-67136 Fussgönheim (DE); PLEISS, Juergen, D-71679 Asperg (DE); SCHWANEBERG, Ulrich, D-71336 Waiblingen (DE); SCHMITT, Jutta, D-70563 Stuttgart (DE); FISCHER, Markus, D-71638 Ludwigsburg (DE); SCHMID, Rolf, D-79329 Stuttgart (DE); LI, Qing-shan, Sakyo-ku Kyoto 606-8502 (JP); APPEL, Daniel, 74348 Lauffen (DE)
(74) Vertreter: Kinzebach, Werner
(86) Internationale Anmeldenummer: PCT/EP2000/007253
(87) Internationale Veröffentlichungsnummer: WO 2001/007630

(56) Entgegenhaltungen:
- WO-A-00/09682
- WO-A-00/31273
- WO-A-01/07574
- DE-A- 19 507 546
- GB-A- 2 294 692
- GB-A- 2 306 485
- US-A- 5 834 297
- MUNRO ANDREW W ET AL: "Regional saturation mutagenesis as an approach to identification of substrate specificity determinants in cytochrome P450 BM3." BIOCHEMICAL SOCIETY TRANSACTIONS, Bd. 21, Nr. 4, 1993, Seite 409S XP000971665 Biochemical Society 647th Meeting on Chromosomal Abnormalities in Cancer Cells: Identification of Molecules Important for Tumour Development;Sheffield, England, UK; July 20-23, 1993 ISSN: 0300-5127
- GRAHAM-LORENCE SANDRA ET AL: "An active site substitution, F87V, converts cytochrome P450 BM-3 into a regio- and stereoselective (14S,15R)-arachidonic acid epoxygenase." JOURNAL OF BIOLOGICAL CHEMISTRY, Bd. 272, Nr. 2, 1997, Seiten 1127-1135, XP002155914 ISSN: 0021-9258
- ENGLAND P A: "The oxidation of naphtalene and pyrene by cytochrome P450cam" FEBS LETTERS,NL,ELSEVIER SCIENCE PUBLISHERS, AMSTERDAM, Bd. 424, Nr. 3, 13. März 1998 (1998-03-13), Seiten 271-274, XP002131695 ISSN: 0014-5793
- JONES ET AL: "The oxidation of polychlorinated benzenes by genetically engineered cytochrome P450cam: potential applications in bioremediation" CHEMICAL COMMUNICATIONS,ROYAL SOCIETY OF CHEMISTRY,GB, Nr. 3, 7. Februar 2000 (2000-02-07), Seiten 247-248, XP002152716 ISSN: 1359-7345
- LI Q S ET AL: "Directed evolution of the fatty-acid hydroxylase P450 BM-3 into an indole-hydroxylating catalyst." CHEMISTRY, (2000 MAY 2) 6 (9) 1531-6. , XP000971670
- GILLAM ELIZABETH M J ET AL: "Formation of indigo by recombinant mammalian cytochrome P450." BIOCHEMICAL AND BIOPHYSICAL RESEARCH COMMUNICATIONS, Bd. 265, Nr. 2, 19. November 1999 (1999-11-19), Seiten 469-472, XP002156034 ISSN: 0006-291X
- CHERRY JOEL R ET AL: "Directed evolution of a fungal peroxidase." NATURE BIOTECHNOLOGY, Bd. 17, Nr. 4, April 1999 (1999-04), Seiten 379-384, XP002156029 ISSN: 1087-0156 in der Anmeldung erwähnt
- SCHWANEBERG ULRICH ET AL: "A continuous spectrophotometric assay for P450 BM-3, a fatty acid hydroxylating enzyme, and its mutant F87A." ANALYTICAL BIOCHEMISTRY, Bd. 269, Nr. 2, 1. Mai 1999 (1999-05-01), Seiten 359-366, XP002156030 ISSN: 0003-2697
- OLIVER CATHERINE F ET AL: "Engineering the substrate specificity of Bacillus megaterium cytochrome P-450 BM3: Hydroxylation of alkyl trimethylammonium compounds." BIOCHEMICAL JOURNAL, Bd. 327, Nr. 2, 1997, Seiten 537-544, XP002156031 ISSN: 0264-6021
- ATKINS W M ET AL: "Molecular recognition in cytochrome P-450: alteration of regioselective alkane hydroxylation via protein engineering" JOURNAL OF THE AMERICAN CHEMICAL SOCIETY,AMERICAN CHEMICAL SOCIETY, WASHINGTON, DC,US, Bd. 111, 1989, Seiten 2715-2717, XP002131692 ISSN: 0002-7863
- SIBBESEN O ET AL: "Putidaredoxin reductase-putidaredoxin-cytochrome P450cam triple fusion protein" JOURNAL OF BIOLOGICAL CHEMISTRY,US,AMERICAN SOCIETY OF BIOLOGICAL CHEMISTS, BALTIMORE, MD, Bd. 271, Nr. 37, 13. September 1996 (1996-09-13), Seiten 22462-22469, XP002131693 ISSN: 0021-9258

## Beschreibung

Die vorliegende Erfindung betrifft Verfahren zur mikrobiologischen Oxidation unterschiedlicher, organischer Substrate, wie N-heterocyclischer aromatischer Verbindungen und insbesondere Verfahren zur Herstellung von Indigo und Indirubin unter Verwendung spezieller Cytochrom P450-Monooxygenasen mit veränderter Substratspezifität, welche zur oxidation organischer Substrate, wie z.B. N-heterocyclischer aromatischer Verbindungen, befähigt sind. Weiterhin betrifft die Erfindung die Verwendung entsprechende kodierender Nukleotidsequenzen, Expressionskonstrukte und Vektoren und transformierte Mikroorganismen zur Durchführung solche Verfahren.

Enzyme mit neuartigen Funktionen und Eigenschaften können entweder durch Screening natürlicher Proben oder durch Protein Engineering bekannter Enzyme bereitgestellt werden. Die letztgenannte Methode kann unter Umständen die geeignetere sein, um Eigenschaften zu induzieren, deren Generierung auf dem Wege natürlicher Selektion unwahrscheinlich ist. Trotz zahlreicher Anstrengungen zum Engineering von Enzymen gibt es bisher nur wenige erfolgreiche Studien zur Förderung der katalytischen Aktivität von Enzymmutanten bezüglich eines bestimmten Substrates (1-10). In diesen bekannten Fällen sind die Substrate strukturell eng verwandt mit dem nativen Substrat des jeweiligen Enzyms. Bisher gibt es keine Berichte über ein erfolgreiches Engineering von Enzymen, welche nach der Modifikation die Umsetzung einer Verbindung katalysieren, welche strukturell völlig verschieden vom nativen Substrat des Enzyms ist.

Die aus dem Bakterium Bacillus megaterium isolierbare Cytochrom P450-Monooxygenase katalysiert gewöhnlich die subterminale Hydroxylierung langkettiger, gesättigter Säuren und der entsprechenden Amide und Alkohole davon oder die Epoxydation ungesättigter langkettiger Fettsäuren oder gesättigter Fettsäuren mit mittlerer Kettenlänge (11-13). Die optimale Kettenlänge gesättigter Fettsäuren beträgt 14 bis 16 Kohlenstoffatome. Fettsäuren mit einer Kettenlänge von weniger als 12 werden nicht hydroxyliert (11).

Die Struktur der Häm-Domäne von P450 BM-3 wurde durch Röntgenstrukturanalyse bestimmt (14-16). Die Substratbindungsstelle liegt in Form einer langen tunnelartigen Öffnung vor, welche von der Moleküloberfläche bis hin zum Häm-Molekül reicht und wird fast ausschließlich von hydrophoben Aminosäureresten begrenzt. Die einzigen geladenen Reste an der Oberfläche der Häm-Domäne sind die Reste Arg47 und Tyr51. Man nimmt an, daß diese an der Bindung der Carboxylatgruppe des Substrates durch Bildung einer Wasserstoffbrückenbindung beteiligt sind (14). Die Mutation von Arg47 zu Glu bewirkt eine Inaktivierung des Enzyms für Arachidonsäure (13), erhöht jedoch dessen Aktivität gegenüber C₁₂-C₁₄-Alkyltrimethylammonium-Verbindungen (17). Eine Substratnutzung für aromatische Verbindungen, insbesondere ein-, zwei- oder mehrkernige, gegebenenfalls heterocyclische, Aromaten, Alkane, Alkene, Cycloalkane und -alkene, wurde für dieses Enzym nicht beschrieben. Es wurde deshalb bisher in der Fachwelt angenommen, daß andere als die bisher beschriebenen organischen Substrate, wie z.B. Indol, aufgrund der deutlichen strukturellen Unterschiede zu den nativen Substraten von P450 BM-3, insbesondere aufgrund des Fehlens funktioneller Gruppen, welche an die oben erwähnten Reste in der Substrattasche binden könnten, keine Substrat darstellen.

Die GB-A-2,294,692 beschreibt Mutanten der Cytochrom P450 Monooxygenase CAM und deren Verwendung zur Oxidation aromatischer Verbindungen.

Es ist deshalb Aufgabe der vorliegenden Erfindung neue Verfahren zur Oxidation organischer Verbindungen unter Verwendung von Cytochrom P450 Monooxygenasen mit veränderter Substratspezifität oder verändertem Substratprofil bereit zu stellen. Insbesondere sollten Monooxygenase-Mutanten eingesetzt werden, welche im Vergleich zu dem nichtmutierten Wildtyp-Enzym mit strukturell deutlich anderen Substraten enzymatisch aktiv sind.

Ein "verändertes Substratprofil" ist für die erfindungsgemäßen Mutanten im Vergleich zum Wildtyp-Enzym zu beobachten. Man beobachtet für die jeweilige Mutante insbesondere eine Verbesserung der Reaktivität, wie z. B. eine Erhöhung der spezifischen Aktivität (ausgedrückt als nmol umgesetztes Substrat/Minute/nmol P450-Enzym), und/oder wenigstens eines kinetischen Parameters, ausgewählt unter Kcat, Km und Kcat/Km (z.B. um mindestens 1 %, wie etwa 10 bis 1000 %, 10 bis 500 %, oder 10 bis 100 %) bei Umsetzung zumindest einer der in den Gruppen a) bis d) definierten oxidierbaren Verbindungen. Die erfindungsgemäße Oxidationsreaktion umfasst die enzymkatalysierte Oxigenierung wenigstens eines exogenen (d.h. dem Reaktionsmedium zugesetzten) oder endo-genen (d.h. im Reaktionsmedium bereits vorhandenen) organischen Substrats. Insbesondere umfasst die erfindungsgemäße Oxidationsreaktion eine Mono- und oder Polyhydroxylierung, wie z.B. eine Mono- und/oder Dihydroxylierung, an einer aliphatischen oder aromatischen C-H-Gruppe, oder eine Epoxidierung an einer vorzugsweise nichtaromatischen C=C-Gruppe. Auch Kombinationen obiger Reaktionen sind denkbar. Das unmittelbare Reaktionsprodukt kann außerdem im Rahmen einer nichtenzymatischen Folge- oder Nebenreaktion weiter umgewandelt werden. Derartige Kombinationen von enzymatischen und nichtenzymatischen Prozessen sind ebenfalls Gegenstand der Erfindung.

Die obige Aufgabe konnte überraschenderweise gelöst werden durch neuartige Verfahren gemäß Anspruch 1.

Insbesondere werden solche Monooxygenasen eingesetzt, deren Substrat-bindender Bereich durch ortsspezifische Mutagenese zur funktionalen Aufnahme neuer, wie z.B. N-heterocyclischer Substrate, befähigt ist.

In einer bevorzugten Ausführungsform der Erfindung sind die verwendeten Monooxygenase löslich, d.h. in nicht-membrangebundener Form existent, und in dieser Form enzymatisch aktiv.

Die erfindungsgemäß verwendeten Monooxygenasen sind vorzugsweise abgeleitet von Cytochrom P450 Monooxygenasen bakteriellen Ursprungs, und zwar abgeleitet von Cytochrom P450 Monooxygenase BM-3 aus Bacillus megaterium mit einer Aminosäuresequenz gemäß SEQ ID NO:2, welche wenigstens eine funktionelle, d.h. die Oxidation neuer organischer Substrate (vgl. insbesondere die im Folgenden definierten Gruppen a) bis d) von Verbindungen), wie z.B. N-heterocyclischer ein-, zwei- oder mehrkerniger armotischer Verbindungen, fördernde Mutation, gemäß der Definition in Anspruch 1. Für die Cytochrom P450 Monooxygenase sind insbesondere die Aminosäuresequenzbereiche 172-224 (F/G-loop-Bereich), 39-43 (β-strand 1), 48-52 (β-strand 2), 67-70 (β-strand 3), 330-335 (β-strand 5), 352-356 (β-strand 8), 73-82 (helix 5) und 86-88 (helix 6) zu nennen.

Die erfindungsgemäß verwendeten Cytochrom P450 Monooxygenase-Mutanten, sind bevorzugt zu wenigstens einer der folgenden Reaktionen befähigt:
a) Oxidation gegebenenfalls substituierter N-, O**-** oder S-heterocyclischer ein-, zwei- oder mehrkerniger armotischer Verbindungen;
b) Oxidation gegebenenfalls substituierter ein- oder mehrkerniger Aromaten;
c) Oxidation geradkettiger oder verzweigter Alkane und Alkene; und
d) Oxidation gegebenenfalls substituierter Cycloalkane und Cycloalkene

In der verwendeten Monooxygenase-Mutanten ist Phe87 ersetzt sein durch eine Aminosäure mit aliphatischer Seitenkette, und zwar Ala, Val, Leu, insbesondere Val; Leu188 kann ersetzt sein durch eine Aminosäure mit Amid-Seitenkette, und zwar Asn oder insbesondere Gln; und Ala74 kann ersetzt sein durch eine andere Aminosäure mit aliphatischer Seitenkette, und zwar Val und insbesondere Gly.

Besonders bevorzugt verwendete Monooxygenase-Mutanten dieses Typs sind, dadurch gekennzeichnet, daß sie wenigstens eine der folgenden ein- oder mehrfachen Aminosäuresubstitutionen aufweist:
1) Phe87Val;
2) Phe87Val, Leu188Gln; oder
3) Phe87Val, Leu188Gln, Ala74Gly.

Der Zahlenwert gibt dabei die Position der Mutation an; vor dem Zahlenwert steht die ursprüngliche, hinter dem Zahlenwert die neu eingeführte Aminosäure.

Erfindungsgemäß oxidierbare Substrate der Gruppe a) sind gegebenenfalls substituierte heterocyclische ein-, zwei- oder mehrkernigen armotischen Verbindungen; insbesondere oxidierbare oder hydroxylierbare N-, O- oder S-heterocyclische ein-, zwei- oder mehrkernige aromatische Verbindungen. Sie umfassen vorzugweise zwei oder drei, insbesondere zwei, vier- bis siebengliedrige, insbesondere sechs- oder fünfgliedrige, kondensierte Ringe, wobei wenigstens einer, vorzugsweise alle Ringe aromatischen Charakter besitzen und wobei wenigstens einer der aromatischen Ringe ein bis drei, vorzugsweise ein N-, O- oder S-Heteroatom im Ring trägt. In der gesamten Ringstruktur können gegebenenfalls ein oder zwei weitere gleiche oder verschiedene Heteroatome enthalten sein. Die aromatischen Verbindungen können weiterhin 1 bis 5 Substituenten an den Ring-Kohlenstoff- oder an den Heteroatomen tragen. Beispiele für geeignete Substituenten sind C₁- bis C₄-Alkyl, wie Methyl, Ethyl, n- oder i-Propyl oder n-, i- oder t- Butyl oder C₂- bis C₄-Alkenyl, wie Ethenyl, 1-Propenyl, 2-Propenyl, 1-Butenyl, 2-Butenyl oder 3-Butenyl, Hydroxyl und Halogen, wie F, Cl, und Br. Die genannten Alkyl- oder Alkenylsubstituenten können gegebenenfalls auch eine Keto- oder Aldehydgruppe aufweisen; Beispiele hierfür sind Propan-2-on-3-yl, Butan-2-on-4-yl, 3-Buten-2-on-4-yl. Nichtlimitierende Beispiele für geeignete heterocyclische Substrate sind insbesondere zweikernige Heterocyclen, wie Indol, N-Methylindol und die mit ein bis drei der oben definierten Substituenten an Kohlenstoffatomen substituierten Analoga davon, wie z.B. 5-Chlor- oder 5-Brom-indol; sowie Chinolin und Chinolinderivate, wie z.B. 8-Methylchinolin, 6-Methylchinolin und Chinaldin; und Benzothiophen und die mit ein bis drei der oben definierten Substituenten an Kohlenstoffatomen substituierten Analoga davon. Außerdem seien genannt dreikernige Heteroaromaten wie Acridin und die mit ein bis drei der oben definierten Substituenten an Kohlenstoffatomen substituierten Analoga davon.

Erfindungsgemäß oxidierbare Substrate der Gruppe b) sind gegebenenfalls substituierte ein- oder mehrkernige, insbesondere ein- oder zweikernige Aromaten, wie Benzol und Naphthalin. Die aromatischen Verbindungen können gegebenenfalls ein oder mehrfach substituiert sein und z.B. 1 bis 5 Substituenten an den Ring-Kohlenstoffatomen tragen. Beispiele für geeignete Substituenten sind C₁ bis C₄-Alkyl, wie Methyl, Ethyl, n- oder i-Propyl oder n-, i- oder t- Butyl, oder C₂ bis C₄-Alkenyl, wie Ethenyl, 1-Propenyl, 2-Propenyl, 1-Butenyl, 2-Butenyl oder 3-Butenyl, Hydroxyl und Halogen, wie F, Cl, und Br. Die genannten Alkyl- oder Alkenylsubstituenten können gegebenenfalls auch eine Keto- oder Aldehydgruppe aufweisen; Beispiele hierfür sind Propan-2-on-3-yl, Butan-2-on-4-yl, 3-Buten-2-on-4-yl. Der Aromat kann gegebenenfalls mit einem vierbis siebengliedrigen, nichtaromatischen Ring kondensiert sein. Der nichtaromatische Ring kann gegebenenfalls eine oder zwei C=C-Doppelbindungen aufweisen, ein- oder mehrfach mit oben genannten Substituenten substituiert sein und gegebenenfalls ein oder zwei Ringheteroatome tragen. Beispiele für besonders brauchbare Aromaten sind einkernige Aromaten, wie Cumol, sowie zweikernige Substrate, wie Inden und Naphthalin, sowie die mit ein bis drei der oben definierten Substituenten an Kohlenstoffatomen substituierten Analoga davon.

Erfindungsgemäß oxidierbare Substrate der Gruppe c) sind geradkettige oder verzweigte Alkane oder Alkene mit 4 bis 15, vorzugsweise 6 bis 12 Kohlenstoffatomen. Als Beispiele können genannt werden n-Butan, n-Pentan, n-Hexan, n-Heptan-, n-Oktan, n-Nonan, n-Decan; n-Undecan und n-Dodecan, sowie die ein- oder mehrfach verzweigten Analoga dieser Verbindungen, wie z.B. analoge Verbindungen mit 1 bis 3 Methyl-Seitengruppen; oder die ein- oder mehrfach, beispielsweise einfach ungesättigten Analoga der oben genannten Alkane.

Erfindungsgemäß oxidierbare Substrate der Gruppe d) sind gegebenenfalls substituierte Cycloalkane und Cycloalkene mit 4 bis 8 Ring-C-Atomen. Beispiele hierfür sind Cyclopentan, Cyclopenten, Cyclohexan, Cyclohexen, Cycloheptan und Cyclohepten. Die Ringstruktur kann dabei ein- oder mehrfach, wie z.B. 1 bis 5 Substituenten gemäß obiger Definition für Verbindungen der Gruppen a) und b) tragen. Nichtlimitierendes Beispiel hierfür sind Ionone, wie α-, β- und γ-Ionon, sowie die entsprechenden Methylionone und Isomethylionone. Besonders bevorzugt sind α- und β-Ionon.

Die Erfindung umfasst auch die Verwendung Nukleinsäuresequenzen, kodierend für eine eine der genannten Monooxygenasen. Solche Nukleinsäuresequenzen sind abgeleitet von SEQ ID NO:1, welche wenigstens eine Nukleotidsubstitution aufweisen, die zu einer der oben beschriebenen funktionellen Aminosäuremutationen führt.

Erfindungsgemäß verwendbar sind auch solche Nukleinsäuresequenzen, die sogenannte stumme Mutationen umfassen oder entsprechend der codon-Nutzung eines speziellen Ursprungs- oder Wirtsorganismus, im Vergleich zu einer konkret genannten Sequenz veränderte sind, ebenso wie natürlich vorkommende Varianten davon. Erfindungsgemäß mit umfasst sind außerdem durch Degeneration des genetischen Codes (d. h. ohne Veränderung der korrespondierenden Aminosäuresequenz) oder konservative Nukleotidsubstitution (d. h. die korrespondierende Aminosäure wird durch eine andere Aminosäure gleicher Ladung, Größe, Polarität und/oder Löslichkeit ersetzt) erhaltene Abwandlungen der Nukleinsäuresequenzen, ebenso wie durch Nukleotidaddition, -insertion, -inversion oder -deletion veränderte Sequenzen, welche für eine erfindungsgemäße Monooxygenase mit "verändertem Substratprofil" kodieren, sowie die korrespondierenden komplemetären Sequenzen.

Gegenstand der Erfindung ist außerdem die Verwendung von Expressionskonstrukten, enthaltend unter der genetischen Kontrolle regulativer Nukleinsäuresequenzen wenigstens eine für eine erfindungsgemäße Mutante kodierende Nukleinsäuresequenz; sowie Vektoren, umfassend wenigstens eines dieser Expressionskonstrukte.

vorzugsweise umfassen die erfindungsgemäß Verwendeten Konstrukte 5'-stromaufwärts von der jeweiligen kodierenden Sequenz einen Promotor und 3'-stromabwärts eine Terminatorsequenz sowie gegebenenfalls weitere übliche regulative Elemente, und zwar jeweils operativ verknüpft mit der kodierenden Sequenz. Unter einer operativen Verknüpfung versteht man die sequentielle Anordnung von Promotor, kodierender Sequenz, Terminator und gegebenenfalls weiterer regulativer Elemente derart, dass jedes der regulativen Elemente seine Funktion bei der Expression der kodierenden Sequenz bestimmungsgemäß erfüllen kann. Beispiele für operativ verknüpfbare Sequenzen sind Targeting-Sequenzen sowie Translationsverstärker, Enhancer, Polyadenylierungssignale und dergleichen. Weitere regulative Elemente umfassen selektierbare Marker, Amplifikationssignale, Replikationaursprünge und dergleichen.

Zusätzlich zu den artifiziellen Regulationssequenzen kann die natürliche Regulationssequenz vor dem eigentlichen Strukturgen noch vorhanden sein. Durch genetische Veränderung kann diese natürliche Regulation gegebenenfalls ausgeschaltet und die Expression der Gene erhöht oder erniedrigt werden. Das Genkonstrukt kann aber auch einfacher aufgebaut sein, das heißt es werden keine zusätzlichen Regulationssignale vor das Strukturgen insertiert und der natürliche Promotor mit seiner Regulation wird nicht entfernt. Statt dessen wird die natürliche Regulationssequenz so mutiert, dass keine Regulation mehr erfolgt und die Genexpression gesteigert oder verringert wird. Die Nukleinsäuresequenzen können in einer oder mehreren Kopien im Genkonstrukt enthalten sein.

Beispiele für brauchbare Promotoren sind: cos-, tac-, trp-, tet-; trp-tet-, lpp-, lac-, lpp-lac-, lacIq-, T7-, T5-, T3-, gal-, trc-, ara-, SP6-, 1-PR- oder im 1-PL-Promotor, die vorteilhafterweise in gram-negativen Bakterien Anwendung finden; sowie die gram-positiven Promotoren amy und SPO2, die Hefepromotoren ADC1, MFa , AC, P-60, CYC1, GAPDH oder die Pflanzenpromotoren CaMV/35S, SSU, OCS, lib4, usp, STLS1, B33, nos oder der Ubiquitin- oder Phaseolin-Promotor. Besonders bevorzugt ist die Verwendung induzierbarer Promotoren, wie z.B. licht- und insbesondere temperaturinduztierbarer Promotoren, wie der PᵣPₗ-Promotor.

Prinzipiell können alle natürlichen Promotoren mit ihren Regulationssequenzen verwendet werden. Darüber hinaus können auch synthetische Promotoren vorteilhaft verwendet werden.

Die genannten regulatorischen Sequenzen sollen die gezielte Expression der Nukleinsäuresequenzen und der Proteinexpression ermöglichen. Dies kann beispielsweise je nach Wirtsorganismus bedeuten, dass das Gen erst nach Induktion exprimiert oder überexprimiert wird, oder dass es sofort exprimiert und/oder überexprimiert wird.

Die regulatorischen Sequenzen bzw. Faktoren können dabei vorzugsweise die Expression positiv beeinflussen und dadurch erhöhen oder erniedrigen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Die Herstellung einer Expressionskassette erfolgt durch Fusion eines geeigneten Promotors mit einer geeigneten Monooxygenase-Nukleotidsequenz sowie einem Terminator- oder Polyadenylierungssignal. Dazu verwendet man gängige Rekombinations- und Klonierungstechniken, wie sie beispielsweise in T. Maniatis, E.F. Fritsch und J. Sambrook, Molecular Cloning: A Laboratory Manual, Cold Spring Harbor Laboratory, Cold Spring Harbor, NY (1989) sowie in T.J. Silhavy, M.L. Berman und L.W. Enquist, Experiments with Gene Fusions, Cold Spring Harbor Laboratory, Cold Spring Harbor, NY (1984) und in Ausubel, F.M. et al., Current Protocols in Molecular Biology, Greene Publishing Assoc. and Wiley Interscience (1987) beschrieben sind.

Das rekombinante Nukleinsäurekonstrukt bzw. Genkonstrukt wird zur Expression in einem geeigneten Wirtsorganismus vorteilhafterweise in einen wirtsspezifischen Vektor insertiert, der eine optimale Expression der Gene im Wirt ermöglicht. Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Viren, wie SV40, CMV, Baculovirus und Adenovirus, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Mit Hilfe solcher Vektoren sind rekombinante Mikroorganismen herstellbar, welche beispielsweise mit wenigstens einem erfindungsgemäßen Vektor transformiert sind und zur Produktion der Mutanten eingesetzt werden können. Vorteilhafterweise werden die oben beschriebenen erfindungsgemäßen rekombinanten Konstrukte in ein geeignetes Wirtssystem eingebracht und exprimiert. Dabei werden vorzugsweise dem Fachmann bekannte geläufige Klonierungs- und Transfektionsmethoden verwendet, um die genannten Nukleinsäuren im jeweiligen Expressionssystem zur Expression zu bringen. Geeignete Systeme werden beispielsweise in Current Protocols in Molecular Biology, F. Ausubel et al., Hrsg., Wiley Interscience, New York 1997, beschrieben.

Als Wirtsorganismen sind prinzipiell alle Organismen geeignet, die eine Expression der erfindungsgemäßen Nukleinsäuren, ihrer Allelvarianten, ihrer funktionellen Äquivalente oder Derivate ermöglichen. Unter Wirtsorganismen sind beispielsweise Bakterien, Pilze, Hefen, pflanzliche oder tierische Zellen zu verstehen. Bevorzugte Organismen sind Bakterien, wie solche der Gattungen Escherichia, wie z. B. Escherichia coli, Streptomyces, Bacillus oder Pseudomonas, eukaryotische Mikroorganismen, wie Saccharomyces cerevisiae, Aspergillus, höhere eukaryotische Zellen aus Tieren oder Pflanzen, beispielsweise Sf9 oder CHO-Zellen.

Gewünschtenfalls kann das Genprodukt auch in transgenen Organismen wie transgenen Tieren, wie insbesondere Mäusen, Schafen oder transgenen Pflanzen zur Expression gebracht werden. Bei den transgenen Organismen kann es sich auch um sogenannte Knock-Out Tiere oder Pflanzen handeln, in denen das korrespondierende endogene Gen ausgeschaltet wurde, wie z. B. durch Mutation oder partielle oder vollständige Deletion.

Die Selektion erfolgreich transformierter Organismen kann durch Markergene erfolgen, die ebenfalls im Vektor oder in der Expressionskassette enthalten sind. Beispiele für solche Markergene sind Gene für Antibiotikaresistenz und für Enzyme, die eine farbgebende Reaktion katalysieren, die ein Anfärben der transformierten Zelle bewirkt. Diese können dann mittels automatischer Zellsortierung selektiert werden. Erfolgreich mit einem Vektor transformierte Mikroorganismen, die ein entsprechendes Antibiotikaresistenzgen (z. B. G418 oder Hygromycin) tragen, lassen sich durch entsprechende Antibiotika-enthaltende Medien oder Nährböden selektieren. Markerproteine, die an der Zelloberfläche präsentiert werden, können zur Selektion mittels Affinitätschromatographie genutzt werden.

Die Kombination aus den Wirtsorganismen und den zu den Organismen passenden Vektoren, wie Plasmide, Viren oder Phagen, wie beispielsweise Plasmide mit dem RNA-Polymerase/Promoter-System, die Phagen λ, µ oder andere temperente Phagen oder Transposons und/ oder weiteren vorteilhaften regulatorischen Sequenzen bildet ein Expressionssystem. Beispielsweise ist unter dem Begriff "Expressionssystem" die Kombination aus Säugetierzellen, wie CHO-Zellen, und Vektoren, wie pcDNA3neo-Vektor, die für Säugetierzellen geeignet sind, zu verstehen.

Wie oben beschrieben, kann das Genprodukt vorteilhaft auch in transgenen Tieren, z. B. Mäusen, Schafen oder transgenen Pflanzen zur Expression gebracht werden. Ebenso ist es möglich, zellfreie Translationssysteme mit der von der Nukleinsäure abgeleiteten RNA zu programmieren.

Der Mikroorganismus kann nach bekannten Verfahren kultiviert und fermentiert werden. Bakterien können beispielsweise in TB- oder LB-Medium und bei einer Temperatur von 20 bis 40 °C und einem pH-Wert von 6 bis 9 vermehrt werden. Im Einzelnen werden geeignete Kultivierungsbedingungen beispielsweise in T. Maniatis, E.F. Fritsch and J. Sambrook, Molecular Cloning: A Laboratory Manual, Cold Spring Harbor Laboratory, Cold Spring Harbor, NY (1989) beschrieben.

Die Zellen werden dann, falls die Monooxygenase nicht in das Kulturmedium sezerniert wird, aufgeschlossen und die Monooxygenase nach bekannten Proteinisolierunqsverfahren aus dem Lysat gewonnen. Die Zellen können wahlweise durch hochfrequenten Ultraschall, durch hohen Druck, wie z. B. in einer French-Druckzelle, durch Osmolyse, durch Einwirkung von Detergenzien, lytischen Enzymen oder organischen Lösungsmitteln, durch Homogenisatoren oder durch Kombination mehrerer der aufgeführten Verfahren aufgeschlossen werden. Eine Aufreinigung der Monooxygenase kann mit bekannten, chromatographischen Verfahren erzielt werden, wie Molekularsieb-Chromatographie (Gelfiltration), wie Q-Sepharose-Chromatographie, Ionenaustausch-Chromatographie und hydrophobe Chromatographie, sowie mit anderen üblichen Verfahren wie Ultrafiltration, Kristallisation, Aussalzen, Dialyse und nativer Gelelektrophorese. Geeignete Verfahren werden beispielsweise in Cooper, F. G., Biochemische Arbeitsmethoden, Verlag Walter de Gruyter, Berlin, New York oder in Scopes, R., Protein Purification, Springer Verlag, New York, Heidelberg, Berlin beschrieben.

Besonders vorteilhaft ist es, zur Isolierung des rekombinanten Proteins Vektorsysteme oder Oligonukleotide zu verwenden, die die cDNA um bestimmte Nukleotidsequenzen verlängern und damit für veränderte Polypeptide oder Fusionsproteine kodieren, die einer einfacheren Reinigung dienen. Derartige geeignete Modifikationen sind beispielsweise als Anker fungierende sogenannte "Tags", wie z. B. die als Hexa-Histidin-Anker bekannte Modifikation oder Epitope, die als Antigene von Antikörpern erkannt werden können (beschrieben zum Beispiel in Harlow, E. and Lane, D., 1988, Antibodies: A Laboratory Manual. Cold Spring Harbor (N.Y.) Press). Diese Anker können zur Anheftung der Proteine an einen festen Träger, wie z. B. einer Polymermatrix, dienen, die beispielsweise in einer Chromatographiesäule eingefüllt sein kann, oder an einer Mikrotiterplatte oder an einem sonstigen Träger verwendet werden kann.

Gleichzeitig.können diese Anker auch zur Erkennung der Proteine verwendet werden. Zur Erkennung der Proteine können außerdem übliche Marker, wie Fluoreszenzfarbstoffe, Enzymarker, die nach Reaktion mit einem Substrat ein detektierbares Reaktionsprodukt bilden, oder radioaktive Marker, allein oder in Kombination mit den Ankern zur Derivatisierung der Proteine verwendet werden.

Die Erfindung betrifft insbesondere ein Verfahren zur mikrobiologischen Oxidation organischer Verbindungen, wie z. B. N-heterocyclischer ein-, zwei- oder mehrkerniger armotischer Verbindungen gemäß obiger Definition, das dadurch gekennzeichnet ist, daß man
a1) einen rekombinanten Mikroorganismus gemäß obiger Definition in einem Kulturmedium in Gegenwart eines exogenen (von außen zugesetzten) oder intermediär gebildeten von der erfindungsgemäßen Monooxygenase oxidierbaren Substrats, vorzugsweise in Gegenwart von Sauerstoff (d.h. aerob), kultiviert; oder
a2) ein Substrat-haltiges Reaktionsmedium mit einem erfindungsgemäßen Enzym, vorzugsweise in Gegenwart von Sauerstoff und einem Elektronendonor, inkubiert; und
b) das gebildete Oxidationsprodukt oder ein Folgeprodukt davon aus dem Medium isoliert.

Der für die Umsetzung erforderliche Sauerstoff gelangt entweder aus der Umgebungsluft in das Reaktionsmedium oder kann, falls erforderlich, in an sich bekannter Weise zugesetzt werden.

Bevorzugt ist das oxidierbare Substrat ausgewählt unter
a) gegebenenfalls substituierten N-heterocyclischen ein-, zwei- oder mehrkernigen armotischen Verbindungen;
b) gegebenenfalls substituierten ein- oder mehrkernigen Aromaten;
c) geradkettigen oder verzweigten Alkanen und Alkenen;
d) gegebenenfalls substituierten Cycloalkanen und Cycloalkenen.

Eine bevorzugte Verfahrensvariante ist auf die Bildung von Indigo/Indirubin gerichtet und dadurch gekennzeichnet, dass das Substrat intermediär in Kultur gebildetes Indol ist und man aus dem Kulturmedium das anfallende Indigo und/oder Indirubin isoliert, welches durch Oxidation von intermediär gebildete Hydroxyindolen erzeugt wurde.

Wird die erfindungsgemäße Oxidation mit einem rekombinanten Mikroorganismus durchgeführt, so erfolgt vorzugsweise zunächst die Kultivierung der Mikroorganismen in Gegenwart von Sauerstoff und in einem Komplexmedium, wie z.B. TB- oder LB- Medium bei einer Kultivierungstemperatur von etwa 20 bis 40 **°**C und einem pH-Wert von etwa 6 bis 9, bis eine ausreichende Zelldichte erreicht ist. Die Zugabe von exogenem Indol ist gewöhnlich nicht erforderlich, da dieses vom Mikroorganismus intermediär gebildet wird. Bei Umsetzung anderer Substrate kann dagegen die Zugabe exogenen Substates erforderlich sein. Um die Oxidationsreaktion besser steuern zu können, bevorzugt man die Verwendung eines induzierbaren, insbesondere temperaturinduzierbaren, Promotors. Man erhöht dabei die Temperatur auf die erforderliche Induktionstemperatur, z.B. 42 °C beim PᵣPₗ-Promotor, behält dies über einen ausreichenden Zeitraum, z. B. 1 bis 10 oder 5 bis 6 Stunden, zur Expression der Monooxygenase-Aktivität bei und verringert anschließend der Temperatur wieder einer Wert von etwa 30 bis 40 °C. Die Kultivierung wird dann in Gegenwart von Sauerstoff 12 Stunden bis 3 Tage fortgesetzt. Insbesondere bei Indol-Oxidation kann der pH-Wert durch Zugabe von NaOH, z.B. auf 9 bis 10, erhöht werden, wodurch die Indigobildung bzw. Indirubinbildung durch Luftoxidation der enzymatisch gebildeten Oxidationsprodukte 2- und 3-Hydroxyindol zusätzlich gefördert wird.

Die erfindungsgemäße Indigo/Indirubin-Bildung wird durch folgendes Reaktionsschema veranschaulicht:

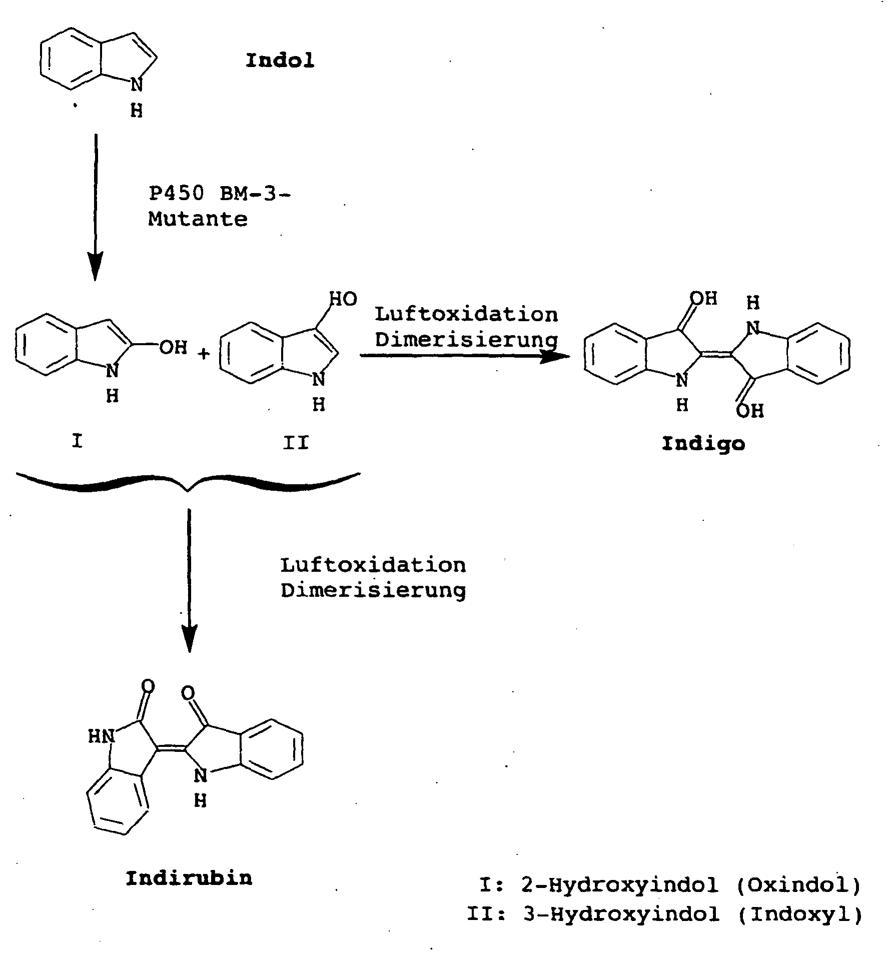

Wird die erfindungsgemäße Oxidation dagegen mit gereinigten oder angereicherten Enzymmutanten durchgeführt so löst man das erfindungsgemäße Enzym in einem exogenes Substrat enthaltenden, wie z.B. Indol enthaltenden, Medium (etwa 0,01 bis 10 mM, oder 0,05 bis 5 mM), und führt die Umsetzung, vorzugsweise in Gegenwart von Sauerstoff, bei einer Temperatur von etwa 10 bis 50 °C, wie z.B. 30 bis 40 °C, und einem pH-Wert von etwa 6 bis 9 (wie z.B. eingestellt mit 100 bis 200 mM Phosphat- oder Tris-Puffer), sowie in Gegenwart eines Reduktionsmittels durch, wobei das Substrat-haltige Medium außerdem bezogen auf das zu oxidierende Substrat einen etwa 1- bis 100-fachen oder 10- bis 100-fachen molaren Überschuß an Reduktionsäquivalenten enthält. Bevorzugtes Reduktionsmittel ist NADPH. Die Zugabe des Reduktionsmittels kann falls erforderlich portionsweise erfolgen.

In analoger Weise werden als oxidierbare Substrate bevorzugt eingesetzt: n-Hexan, n-Octan, n-Decan, n-Dodecan, Cumol, 1-Methylindol, 5-Cl- oder Br-Indol, Inden, Benzothiophen, α-, β- und γ-Ionon, Acridin, Naphthalin, 6-Methyl- oder 8-Methylchinolin, Chinolin und Chinaldin.

Beispielsweise kann die erfindungsgemäße enzymatische Oxidationsreaktion unter folgenden Bedingungen durchgeführt werden:

| | |
|---|---|
| Substratkonzentration: | 0,01 bis 20 mM |
| Enzymkonzentration: | 0,1 bis 10 mg/ml |
| Reaktionstemperatur: | 10 bis 50 °C |
| pH: | 6 bis 8 |
| Puffer: | 0,05 bis 0,2 M Kaliumphosphat,oder Tris/ HCl |
| Elektronendonor: | wird bevorzugt portionsweise zugegeben (Anfangskonzentration etwa 0,1 bis 2 mg/ml) |

Vor dem Start der Reaktion, z.B. durch Zugabe der Elektronendonors (z.B. NADPH) kann kurzzeitig (1 bis 5 Minuten) vorinkubiert werden (bei etwa 20 bis 40 °C). Die Umsetzung erfolgt aeorb, gegebenenfalls bei zusätzlicher Einleitung von Sauerstoff.

Beim erfindungsgemäßen Substratoxidationsprozess wird im Reaktionsmedium enthaltener oder zugesetzter Sauerstoff reduktiv enzymatisch gespalten. Die erforderlichen Reduktionsäquivalente werden von dem zugesetzten Reduktionsmittel (Elektronendonor) zur Verfügung gestellt.

Das gebildete Oxidationsprodukt kann dann in herkömmlicher Weise, wie z.B. durch Extraktion oder Chromatographie, vom Medium abgetrennt und gereinigt werden.

Weitere Gegenstände der Erfindung betreffen Bioreaktoren, umfassend ein erfindungsgemäßes Enzym oder einen erfindungsgemäßen rekombinanten Mikroorganismus in immobilisierter Form.

Ein letzter Gegenstand der Erfindung betrifft die Verwendung einer erfindungsgemäßen Cytochrom P450 Monooxygenase oder eines erfindungsgemäßen Vektors oder Mikroorganismus zur mikrobiologischen Oxidation eines Substrates aus einer der Gruppen a) bis d), insbesondere N-heterocyclischer ein-, zwei- oder mehrkerniger armotischer Verbindungen, und bevorzugt zur Bildung von Indigo und/ oder Indirubin.

Die vorliegende Erfindung wird nunmehr unter Bezugnahme auf die folgenden Beispiele näher beschrieben.

### Beispiel 1:

### Randomisierung spezieller Codons von P450 BM-3

Die Versuche wurden im wesentlichen wie beschrieben in (19) durchgeführt. Drei Positionen (Phe87, Leu188 und Ala74) wurden mit Hilfe von ortsspezifischer Mutagenese unter Verwendung des Stratagene QuikChange kit (La Jolla, CA, USA) randomisiert. Folgende PCR-Primer wurden für die einzelnen Positionen verwendet:
Phe87: -5'-gcaggagacgggttgnnnacaagctggacg-3' (SEQ ID NO:3),
   5'-cgtccagcttgtnnncaacccgtctcctgc-3', (SEQ ID NO:4)
Leu188: 5'-gaagcaatgaacaagnnncagcgagcaaatccag-3' (SEQ ID NO:5),
   5'-ctggatttgctcgctgnnncttgttcattgcttc-3' (SEQ ID NO:6);
Ala74: 5'-gctttgataaaaacttaaagtcaannncttaaatttgtacg-3' (SEQ ID No:7),
   5'-cgtacaaatttaagnnnttgacttaagtttttatcaaagc-3' (SEQ ID NO:8)

Die Bedingungen für die PCR waren für alle drei Positionen identisch. Insbesondere wurden je 50 µl Reaktionsvolumen 17,5 pmol eines jeden Primers, 20 pmol Template-Plasmid-DNA, 3 U der Pfu Polymerase und 3,25 nmol von jedem dNTP verwendet. Die PCR Reaktion wurde bei 94°C /1 min gestartet und dann wurde folgender Temperaturzyklus 20 mal durchgeführt: 94°C, 1 min; 46°C, 2,5 min; 72°C, 17 min. Nach 20 Zyklen wurde die Reaktion 15 min bei 72°C fortgesetzt. Nach der PCR wurde die Template DNA mit 20 U DpnI bei 37°C 3 h verdaut. Anschließend wurde E. coli DH5α transformiert. Die transformierten E. coli DH5α-Zellen wurden auf LB-Agarplatten ausplattiert, welche 150 µg/ml Ampicillin enthielten. Anschließend wurde 18 h bei 37°C inkubiert.

### Beispiel 2:

### Expression und Reinigung der P450 BM-3 und dessen Mutanten und Produktion eines blauen Pigmentes

Das P450 BM-3-Gen und die Mutanten davon wurden unter der Kontrolle des starken, Temperatur-induzierbaren P_{R}P_{L}-Promotors des Plasmids pCYTEXP1 in E. coli DH5α wie bereits beschrieben (20), exprimiert. Kolonien wurden mit sterilen Zahnstochern aufgenommen und in Mikrotiterplatten mit 96 Vertiefungen, enthaltend je Vertiefung 200 µl TB-Medium und 100 µg/ml Ampicillin transferiert. Anschließend wurde über Nacht bei 37°C inkubiert. 40 µl der Zellkultur einer jeden Vertiefung wurden anschließend in ein Kulturröhrchen überführt, das 2 ml TB-Medium mit 100 µg/ml Ampicillin enthält. Anschließend wurde 2 h bei 37°C kultiviert. Dann wurde die Tempertur zur Induktion 6 h auf 42°C erhöht. Dann wurde die Kultivierung über Nacht bei 37°C fortgesetzt, wobei ein blaues Pigment produziert wurde.

Die präparative Herstellung von Enzym oder blauem Pigment wurde ausgehend von einer 300 ml Zellkultur (OD₅₇₈ₙₘ = 0, 8 bis 1,0) durchgefürht. Zur Isolierung des Enzymes wurden die Zellen 10 min bei 4000 Upm abzentrifugiert, in 0,1 M KₓPO₄-Puffer, pH 7,4 resuspendiert. Die eisgekühlten Zellen wurden mit Hilfe eines Branson Sonifiers W25 (Dietzenbach, Deutschland) bei einer Energieoutput von 80 w durch dreimalige Beschallung von 2 min vorsichtig aufgeschlossen. Die Suspensionen wurden 20 min bei 32570 x g zentrifugiert. Der Rohextrakt wurde zur Aktivitätsbestimmung bzw. zur Enzymreinigung eingesetzt. Die Enzymreinigung erfolgte wie in (21) bereits beschrieben, worauf hiermit ausdrücklich Bezug genommen wird. Die Konzentration an gereinigtem Enzym wurde über die Extinktionsdifferenz bei 450 und 490 nm, wie in (11) bereits beschrieben, unter Verwendung eines Extinktionskoeffizienten ε von 91 mM⁻¹ cm⁻¹ bestimmt.

### Beispiel 3:

### Isolierung von Mutanten, welche große Mengen an blauem Pigment produzieren

Jeweils 100 Kolonien wurden von den Mutanten einer jeden Position isoliert, welche durch randomisierte Mutagenese des Codons der entsprechenden Position erzeugt wurden. Diese Kolonien wurden in Kulturröhrchen zur Produktion von blauem Pigment kultiviert. Nach dem Waschen der Zellen mit Wasser und mehreren langsamen Zentrifugationsschritten (500 Upm) wurde das blaue Pigment mit Dimethylsulfoxid (DMSO) extrahiert. Die Löslichkeit des blauen Pigmentes war in DMSO am größten. Die Absorption des Extraktes wurde bei 677 nm bestimmt. Diejenige Mutante, welche die größte Menge an blauem Pigment von allen Mutanten einer bestimmten Position produzierte, wurde für eine DNA-Sequenzierung (ABI DNA Sequenzierungs-Kit; ABI Prism^{™} 377 DNA Sequencer) verwendet und außerdem als Template für ortsspezifische randomisierte Mutagenese verwendet.

### Beispiel 4:

### Aktivitätstest für die Indol-Hydroxylierung

Die Indol-Hydroxylierungsaktivität wurde in einer Lösung getestet, die 8 µl einer 10-500 mM Indollösung in DMSO, 850 µl Tris/ HCl-Puffer (0,1 M, pH 8,2) und 0,6 nmol P450 BM-3 Wildtyp oder Mutante in einem Endvolumen von 1 ml enthielt. Das Gemisch wurde 9 min vorinkubiert, bevor man die Reaktion durch Zugabe von 50 µl einer wässrigen 1 mM Lösung von NADPH startete. Die Reaktion wurde nach 20 sec durch Zugabe von 60 µl 1,2 M KOH gestoppt. Innerhalb von 5 bis 30 sec (unter aeroben Bedingungen) wurden die Enzymprodukte vollständig in Indigo [Δ^{2,2'}-Biindolin]-3,3'-dion) und Indirubin ([Δ^{2,3'}-Biindolin]-2',3-dion) überführt. Die Indigoproduktion wurde über dessen Absorption bei 670 nm bestimmt. Eine Eichkurve mit reinem Indigo zeigte einen Extinktionskoeffizienten von 3,9 mm⁻¹ cm⁻¹ bei dieser Wellenlänge. Ein linearer Kurvenverlauf wurde für die Indigoproduktion in einer Reaktionszeit von 40 sec unter Verwendung von 0,6 nmol Wildtyp bzw. P450 BM-3-Mutante und 0,05 bis 5,0 mM Indol erhalten. Indirubin zeigt eine sehr schwache Absorption bei 670 nm und die gebildete Indirubinmenge war sehr viel geringer als die gebildete Indigomenge. Die Bildung von Indirubin wurde bei der Bestimmung der kinetischen Parameter vernachlässigt. Der NADPH-Verbrauch wurde bei 340 nm bestimmt und unter Verwendung eines Extinktionskoeffizienten von 6,2 mM⁻¹ cm⁻¹ wie beschrieben (17) berechnet.

### Beispiel 5:

### Reinigung von Indigo und Indirubin

Nach waschen der Zellen mit Wasser und wiederholter Zentrifugation bei 500 g wurde das gebildete blaue Pellet mit Tetrahydrofuran (THF) extrahiert. Der Extrakt wurde bis fast zur Trockene eingedampft und das rote Pigment wurde mehrmals mit 50 ml absolutem Ethanol extrahiert. Der verbleibende blaue Feststoff wurde in THF gelöst und durch Dünnschichtchromatographie (TLC) analysiert. Die Ethanollösung wurde eingedampft und durch Silicagelchromatographie (DC 60, Merck, Darmstadt, Deutschland; 2 cm x 30 cm) gereinigt, bevor sie mit THF und Petrolether in einem Verhältnis von 1:2 gewaschen wurde. Die erhaltene rote Lösung wurde eingedampft und deren Reinheit wurde durch TLC bestimmt. Die Absorptionsspektren des blauen und des roten Pigmentes wurden mit Hilfe eines Ultraspec 3000 Spektrophotometers (Pharmacia, Uppsala, Sweden) in einem Bereich von 400 bis 800 nm bestimmt. Außerdem wurde der blaue und der rote Farbstoff durch Massenspektrometrie und ¹H-NMR Spektroskopie analysiert.

### Versuchsergebnisse

### 1. Erhöhung der Produktivität für blaues Pigment durch P450 BM-3-Mutagenese

Natives P450 BM-3 besitzt nicht die Fähigkeit zur Produktion des blauen Indigo-enthaltenden Pigments, bzw. der Vorläufersubstanten 2- bzw 3-Hydroxyindol. Um eine ausreichende Menge an blauem Pigment herstellen zu können, wurde P450 BM3 einer gezielten Evolution ausgesetzt. Sämtliche Mutanten, welche das blaue Pigment produzierten, wurden sequenziert. Es wurde festgestellt, daß wenigstens eine der folgenden drei Positionen mutiert waren: phe87, Leu188 und Ala74. Es wurde deshalb angenommen, daß diese drei Positionen eine entscheidende Rolle für die Aktivität von P450 BM-3 bei der Produktion von blauem Pigment spielen. Aus der Struktur der Häm-Domäne von Cytochrom-P450-BM 3, komplexiert mit Palmitoleinsäure sieht man, daß Phe87 das Substrat an einem näheren Heranrücken an die Häm-Gruppe hindert (14). Die Mutante Phe87Val zeigt eine hohe Regio- und Stereoselektität bei der Epoxidation von (14S, 15R)-Arachidonsäure (13) und die Mutante Phe87Ala verschiebt die Hydroxylierungsposition von ω-1, ω-2 und ω-3 zu (22). Die Position 87 wurde deshalb als erste für die ortspezifische randomisierte Mutanese mit Hilfe von PCR ausgewählt. In Röhrchenkulturen wurden 7 Kolonien erhalten, welche eine geringe Menge an blauem Pigment nach Induktion produzierten. Die Kolonie, welche die größte Menge des blauen Pigments produzierte, wurde für die DNA-Sequenzierung ausgewählt. Die Sequenzdaten ergaben eine Substitution von Phe87 durch Val. Die Mutante Phe87Val wurde anschließend als Template für die zweite Runde der ortsspezifischen randomisierte Mutagenese an Position Leu188 verwendet. Die Struktur der Häm-Domäne, komplexiert mit Palmitoleinsäure zeigt, daß die Repositionierung der F- und G-Helices den Rest Leu188 in direkten Kontakt mit dem Substrat bringt (14). Diese Position könnte deshalb eine wichtige Rolle bei der Substratbindung oder -orientierung spielen. Nach dem zweiten Screeningdurchgang wurden 31 Kolonien beobachtet,' welche das blaue Pigment produzierten. Die Mutante, welche die größte Pigmentmenge produzierte, enthielt die Substitutionen Phe87Va1 und Leu188Gln. Diese Mutante wurde anschließend in Position Ala74 im dritten Durchgang der ortspezifischen randomisierten Mutagenese mutiert. Man erhielt dabei die Dreifachmutante F87L188A74 (Phe87Val, Leul88Gln und Ala74Gly), welche mehrere mg blaues Pigment in einem 2-Liter-Kolben, enthaltend 300 ml TB-Medium, produzierte. Diese Menge reichte zur Isolierung und Charakterisierung des blauen Pigmentes aus.

### 2. Isolierung und Identifizierung des blauen Pigments

Nach dem Auswaschen der Zellen wurde das verbleibende blaue Pellet mit THF extrahiert und TLC analysiert. Das blaue Pigment wurde in eine schneller wandernde blaue Komponente und in eine langsamer wandernde rote Komponente aufgetrennt. Beide Komponenten zeigten exakt die gleichen Mobilitätsparameter wie die Komponenten einer kommerziellen Indigo-Probe.

Nach der Reinigung wurden die Absorptionsspektra beider Komponenten in DMSO bestimmt. Die blaue Komponente zeigte das gleiche Spektrum wie eine kommerzielle Indigoprobe. Die gereinigte blaue und rote Komponente wurden jeweils durch Massenspektrometrie analysiert. Die Massenspektra beider Pigmente zeigten einen starken Molekülionenpeak bei m/z = 262 und zwei Fragmentionenpeaks bei m/z = 234 und 205 (relative Intensität jeweils 10%). Dieses Muster ist typisch für indigoide Verbindungen. Die Elementarzusammensetzung dieser Ionen wurde durch hochauflösende Massenspektrometrie bestimmt als C₁₆H₁₀N₂O₂, C₁₅H₁₀N₂0 bzw. C₁₄H₉N₂. Dies ist ebenfalls charakteristisch für Strukturen vom Indigotyp. Das blaue Pigment wurde somit als Indigo und das rote Pigment als Indirubin bestimmt. Zur Bestätigung der Struktur wurden 500 MHz ¹H-NMR-Spektren beider Pigmente in DMSO-D₆-Lösung durchgeführt. Die Ergebnisse stimmten mit den Literaturangaben (23) überein.

### 3. Produktion von Indigo mit isolierten Enzymen

Es ist bekannt, daß Indigo aus Indol durch mikrobielle Transformation zugänglich ist (24-26). Keines dieser mikrobiellen Systeme enthielt jedoch eine P450 Monooxygenase. Erfindungsgemäß wurde zunächst die katalytische Aktivität des reinen Enzyms für Indol bestimmt. Die Mutante F87L188A74 wurde mit Indol vermischt. Keine Farbreaktion war zu beobachten. Erst nach Zugabe von NADPH zum Reaktionsgemisch bildete sich das blaue Pigment nach etwa 20 min. Durch Einstellung des pH-Werts der Reaktionsmischung auf einen Wert von etwa 11, 30 sec nach Zugabe von NADPH, wurde die blaue Färbung innerhalb von wenigen Sekunden sichtbar. Kontrollversuche unter Verwendung von nativem P450 BM-3 waren immer negativ, selbst unter Verwendung erhöhter Konzentrationen an Enzym, Indol und NADPH. Das blaue Pigment wurde mit Ethylacetat extrahiert und durch TLC analysiert. Das blaue Pigment trennte sich wieder in eine schneller laufende blaue Komponente und in eine langsamer laufende rote Komponente auf. Die Rf-Werte und die Absorptionsspektren waren identisch mit denjenigen Werten der Extakte aus der Fermentationsbrühe. Die F87L188A74-Mutante von P450 BM-3 stellt somit eine Indolhydroxylase dar.

Es sind bisher zwei Wege für die enzymatische Transformation von Indol zu Indigo beschrieben worden. Ein Weg wird durch eine Dioxygenase, der andere durch eine StyrolMonooxygenase katalysiert (24,25). Die NADPH-Stöchiometrie beträgt in beiden Fällen 2. Es wurde deshalb angenommen, daß im Gegensatz zu den Dioxygenasen die erfindungsgemäße Mutante F87L188A74 Indol in nur einer Position hydroxyliert, um Oxindol (2-Hydroxyindol) oder Indoxyl (3-Hydroxyindol) zu bilden.

### 4. Kinetische Parameter der Indolhydroxylierung

Reine Proben des Wildtyp-Enzyms P450 BM-3 und der Mutanten Leu188G1n, Phe87Val, F87L188 und F87L188A74 wurden zur Bestimmung der kinetischen Parameter der Indolhydroxylierung verwendet. Die Ergebnisse sind in folgender Tabelle 1 zusammengefaßt.

**Tabelle 1: Kinetische Parameter der P450 BM-3 Mutanten für Indolhydroxylierung**

| Mutanten | K_{cat} (S⁻¹) | | Kₘ(mM) | | K_{cat}/Kₘ(M⁻¹s⁻¹) |
|---|---|---|---|---|---|
| WT | -^{a)} | | - | | - |
| Leu188Gln | n.d.^{b)} | | n.d. | | n.d. |
| Phe87Val | 2,03 | (0,14) | 17,0 | (1,0) | 119 |
| F87L188 | 2,28 | (0,16) | 4,2 | (0,4) | 543 |
| F87L188A74 | 2,73 | (0,16) | 2,0 | (0,2) | 1365 |

| | | | | | |
|---|---|---|---|---|---|
| ^{a)} keine Aktivität wurde beobachtet; ^{b)} nicht bestimmt (Aktivität war zu gering um gemessen zu werden | | | | | |

Selbst beim Überschuß an gereinigtem Enzym und hoher Indolkonzentration ist das Wildtyp-Enzym nicht in der Lage, Indol zu oxidieren. Die Mutante Leu188Gln zeigt eine geringe Aktivität. Die Mutante Phe87Val zeigt eine katalytische Wirksamkeit von 119 M⁻¹s⁻¹ für die Indolhydroxylierung. Die katalytische Effizienz der Doppelmutante F87L188 (Phe87Val,Leu188Gln) erhöhte sich auf 543 M⁻¹s⁻¹ und wurde durch Einführung der weiteren Substitution Ala74Gly auf 1365 M⁻¹s⁻¹ erhöht. Die K_{cat}-Werte erhöhten sich von Phe87Val zur Dreifachmutante hin um insgesamt 35%, während die Kₘ-Werte etwa um das Siebenfache abnahmen. Dies weist darauf hin, daß Ala74Gly und Leu188Gln vorwiegend an der Substatbindung beteiligt sind.

Die Indol-Turnover-Rate (K_{cat}=2,73 s-¹) war für die Dreifachmutante F87L188A74 mehr als zehnfach höher als für die meisten P450-Enzyme (18).

### Beispiel 6:

### n-Oktanhydroxylierung mit modifizierter Cytochrom P450 Monooxygenase

Die Umsetzungen wurden mit einer P450 BM-3-Monooxygenase Mutante durchgeführt, die folgende Mutationen enthält: Phe87Val Leu188Gln Ala74Gly

Als Substrat wurde n-Octan gewählt. Für die Hydroxylierung des n-Octans wurde folgender aerober Reaktionsansatz verwendet:

| | |
|---|---|
| P450 BM-3 Mutante: | 17,5 mg (Lyophylisat) |
| Reaktionspuffer: | 9,1 ml (Kaliumphosphat-Puffer 50 mM, pH 7.5) |
| Substrat: | 50 µl einer 60 mM Lösung (in Aceton) |
| Temperatur: | 25°C |

Enzymlyophylisat wurde in 500 µl Reaktionspuffer gelöst und zunächst mit Substrat und Reaktionspuffer 5 min bei Raumtemperatur inkubiert. Anschließend erfolgte die Zugabe von 300 µl NADPH-Lösung (5mg/ml). Die NADPH Zugabe wurde noch zweimal wiederholt. Der Reaktionsverlauf wurde durch Absorptionsmessungen bei 340 nm verfolgt, wobei die NADPH Abnahme beobachtet werden kann. NADPH wird dabei in 300 µl-Schritten zugegeben, da eine zu hohe Konzentration an NADPH in der Reaktionslösung zu einer Inaktivierung des Enzyms führt. Zur Isolierung der Produkte wurde anschließend die Reaktionslösung 3 mal mit 5 ml Diethylether extrahiert. Die vereinigten organischen Phasen wurden über MgSO₄ getrocknet und eingeengt. Anschließend wurden die Produkte über DC, GC/MS und NMR charakterisiert.

Die GC/MS Analyse des Reaktionsgemisches führte zu folgendem Ergebnis:

| Verbindung | Rt[min]¹⁾ | Umsatz [%] |
|---|---|---|
| 4-Octanol | 13.51 | 37 |
| 3-Octanol | 14.08 | 47 |
| 2-Octanol | 14.26 | 16 |

| | | |
|---|---|---|
| ¹⁾Temperaturprogramm: 40°C 1 min isotherm / 3°C/min 95°C /10°C/min 275°C; Apparatur: Finnigan MAT 95; GC: HP 5890 Series II Split Injector; Säule: HP-5MS (Methylsiloxan) 30m x 0.25mm; Trägergas:0,065 ml/min He | | |

Edukt wurde nicht mehr gefunden.

### Beispiel 7:

### Hydroxylierung von Aromaten, Heteroaromaten und Trimethylcyclohexenylverbindüngen

a) Beispiel 6 wurde wiederholt, wobei jedoch anstelle von n-Oktan als Substrat Naphthalin eingesetzt wurde. Als Produkte wurden 1-Naphthol und cis-1,2-Dihydroxy-1,2-dihydronaphthalin identifiziert. Vom eingesetzten Naphthalin wurden 88% umgesetzt.
   Analytik für Umsetzungen mit Naphthalin
   GC:
   Apparatur: Carlo Erba Strumentazion Typ HRGC 4160 on Column Injector; Säule:DB5 30m x 0,2 mm; Material: 5% Diphenyl- 95% Dimethylpolysiloxan; Trägergas:0,5 bar H₂;
   Temperaturprogramm: 40°C 1 min isotherm / 10°C/min bis 300°C Rt(1-Naphthol) = 16.68
   NMR:
   Im ¹H-NMR konnte 1-Naphthol und cis-1,2-Dihydroxy-1,2-dihydronaphthalin identifiziert werden.
b) Beispiel 6 wurde wiederholt, wobei jedoch anstelle von n-Oktan als Substrat 8-Methylchinolin eingesetzt wurde. Als Hauptprodukt wurde 5-Hydroxy-8-methylchinolin neben weiteren Derivaten (Produktverhältnis 5:1) identifiziert. Vom eingesetzten Edukt wurden 35% umgesetzt.
c) Beispiel 6 wurde wiederholt, wobei jedoch anstelle von n-Oktan als Substrat α-Ionon eingesetzt wurde. Als Hauptprodukt wurde 3-Hydroxy-α-ionon neben weiteren Derivaten (Produktverhältnis 76:24) identifiziert. Vom eingesetzten Edukt wurden 60 % umgesetzt.
d) Beispiel 6 wurde wiederholt, wobei jedoch anstelle von n-Oktan als Substrat Cumol (i-Propylbenzol) eingesetzt wurde. Es wurden fünf Monohydroxyprodukte und ein Dihydroxyprodukt identifiziert. Vom eingesetzten Edukt wurden 70% umgesetzt.

### LITERATUR

1. Yano, T., Oue, S., and Kagamiyama, H. (1998) Proc. Natl. Acad Sci. USA 95,5511-5515.
2. Zhang, J.-H., Dawes, G., and Stemmer, W. P. C. (1997) Proc. Natl. Acad Sci. USA 94, 4504-4509.
3. Wan, L., Twitchett, M. B., Eltis, L. D., Mauk, A. G., and Smith, M. (1998) Proc. Natl. Acad Sci USA 95,12825-12831.
4. Cronin, C. N. (1998) J. Biol. Chem 273,24465-24469.
5. Wilks, H. M., Hart, K- W., Feeney, R., Dunn, C. R., Muirhead, H., Chia, W. N., Barstow, D. A., Atkinson, T., Clarke, A. R., Holbrook, I J. (1988) Science 242, 1541-1544.
6. Hedstrom, L., Szilagyi, L., Rutter, W. J. (1992) Science 255, 1249-1253.
7. Tucker, C. L., Hurley, J. H., Miller, T. R., and Hurley, I B. (1998) Proc. Natl. Acad Sci. USA 95, 5993-5997.
8. Quemeneur, E., Moutiez, J.-B. C., and Menez, A. (1998) Nature (London) 391, 301-303.
9. Marsden, A- F. A., Wilkinson, B., Cortes, J., Dunster, N. J., Staunton, I Leadlay, P. F. (1998) Science 279, 199-201.
10. Chen, R., Greer, A., and Dean, A. M. (1998) Proc. Natl. Acad Sci. US4 95, 11666-11670.
11. Boddupalli, S. S., Estabrook, R. W. and Peterson, J. A. (1990) J Biol. Chem 265, 4233-4239.
12. Capdevila, J. H., Wie, S., Helvig, C., Falck, J. R., Belosludtsev, Y.,Truan, G., Graham-Lorence, S. E., and Peterson, J. A. (1996) J. Biol. Chem 271, 22663-22671.
13. Graham-Lorence, S., Truan, G., Peterson, J. A., Flack, J. R., WeL S., Helvig, C., Capdevilla, J. H. (1997) J. Biol. Chem 272,1127-1135.
14. Li, H., Poulos, T. L. (1997) Nat. Structural Biol., 4, 140-146.
15. Ravichandran, K G., Sekhar, S., Boddupalli, S., Hasemann, C. A., Peterson, J. A., Deisenhofer, 1 (1993) Science 261,731-736.
16. Modi S., Sutcliffe, M. J., Primrose, W. U., Lian, L.- Y., Roberts, G. C. K (1996) Nat. Structural Biol. 3, 414-417.
17. Oliver, C.F., ModL S., Primrose, W.U., Lian, L.Y. and Roberts, G.C.K (1997) Biochem. J 327,537-544.
18. Guengerich, F.G. (1991) J. Biol. Chem 266,10019-10022.
19. Cherry, J. R., Lamsa, M. H., Schneider, P., Vind, J., Svendsen, A-, Jones, A., and Pedersen, A. H. (1999) Nature Biotechnology 17, 379-384.
20. Schwaneberg, U., Schmidt-Dannert, C., Schmitt, J., and Schmid, R. D. (1999) Anal Biochem. 269,359-366.
21. Schwaneberg, U, Sprauer, AL, Schmidt-Dannert, C., and Schmid, R. D. J of Chromatogr. A, in press.
22. Oliver, C.F., Modi, S., Sutcliffe, M.J., Primrose, W.U., Lian, L.Y. and Roberts, G.C.K (1997) Biochemistry 36, 1567-1572.
23. Hart, S., Koch, KR., and Woods, D.R. (1992) J Gen. Microbiol. 138,211-216
24. Murdock, D., Ensley, B.D., Serdar, C. and Thalen, M. (1993) Bio/Technology 11, 381-385.
25. O'Connor, ICE., Dobson, A-W. and Hartmans, S. (1997) Appl. Environ. Microbiol. 63, 4287-4291.
26. Eaton, R. W. and Chapman, P. J. (1995) J Bacteriol. 177, 6983-6988.

## Patentansprüche

1. Verfahren zur mikrobiologischen Oxidation einer N-, O- oder S-heterocyclischen ein- oder mehrkernigen aromatischen Verbindung, **dadurch gekennzeichnet, dass** man
a1) einen rekombinanten Cytochrom P450 produzierenden Mikroorganismus, transformiert mit einem Vektor, enthaltend wenigstens ein Expressionskonstrukt, das unter der genetischen Kontrolle regulativer Nukleinsäuresequenzen eine kodierende Sequenz für Monooxygenase trägt, in einem Kulturmedium, in Gegenwart eines exogenen oder intermediär gebildeten Substrats, kultiviert; oder
a2) ein Substrat-haltiges Reaktionsmedium mit einer Cytochrom P450 Monooxygenase bakteriellen Ursprungs inkubiert; und
b) das gebildete Oxidationsprodukt oder ein Folgeprodukt davon aus dem Medium isoliert;
wobei die Monooxygenase abgeleitet ist von Cytochrom P450 Monooxygenase BM-3 aus Bacillus megaterium mit einer Aminosäuresequenz gemäß SEQ ID NO:2, welche eine funktionelle Mutation in der Aminosäuresequenzposition 87; oder in den Positionen 87 und 188; oder in den Positionen 87, 188 und 74 aufweist, wobei
Phe87 ersetzt ist durch Ala, Val oder Leu;
Leu188 ersetzt ist durch Asn oder Gln; und
Ala 74 ersetzt ist durch Val oder Gly.

2. Verfahren nach Anspruch 1, **dadurch gekennzeichnet, dass** das exogene oder intermediär gebildete Substrat ausgewählt ist unter gegebenenfalls substituierten N- , O oder S-heterocyclischen ein- oder mehrkernigen aromatischen Verbindungen.

3. Verfahren zur mikrobiologischen Oxidation einer Verbindung ausgewählt unter gegebenenfalls substituierten ein- oder mehrkernigen Aromaten; geradkettigen oder verzweigten Alkane und Alkene; und gegebenenfalls substituierten Cycloalkanen und Cycloalkenen;
**dadurch gekennzeichnet, dass** man
a1) einen rekombinanten, Cytochrom P450 produzierenden Mikroorganismus, transformiert mit einem Vektor, enthaltend wenigstens ein Expressionskonstrukt, das unter der genetischen Kontrolle regulativer Nukleinsäuresequenzen eine kodierende Sequenz für Monooxygenase trägt, in einem Kulturmedium, in Gegenwart eines exogenen oder intermediär gebildeten Substrats, kultiviert; oder
a2) ein Substrat-haltiges Reaktionsmedium mit einer Cytochrom P450 Monooxygenase inkubiert; und
b) das gebildete Oxidationsprodukt oder ein Folgeprodukt davon aus dem Medium isoliert;
wobei die Monooxygenase abgeleitet ist von Cytochrom P450 Monooxygenase BM-3 aus Bacillus megaterium mit einer Aminosäuresequenz gemäß SEQ ID NO:2, welche eine funktionelle Mutation in der Aminosäuresequenzposition 87; oder in den Positionen 87 und 188; oder in den Positionen 87, 188 und 74 aufweist, wobei
Phe87 ersetzt ist durch Ala, Val oder Leu;
Leu188 ersetzt ist durch Asn oder Gln; und
Ala 74 ersetzt ist durch Val oder Gly.

4. Verfahren nach Anspruch 3, **dadurch gekennzeichnet, dass** das exogene oder intermediär gebildete Substrat ausgewählt ist unter:
gegebenenfalls substituierten ein- oder mehrkernigen Aromaten;
geradkettigen oder verzweigten Alkanen und Alkenen; und
gegebenenfalls substituierten Cycloalkanen und Cycloalkenen.

5. Verfahren nach einem der vorhergehenden Ansprüche, **dadurch gekennzeichnet, dass** man als exogenes Substrat wenigstens eine Verbindung, ausgewählt unter den oben definierten Gruppen von Verbindungen, einem Medium zusetzt und die Oxidation durch enzymatische Umsetzung des substrat-haltiges Mediums in Gegenwart von Sauerstoff bei einer Temperatur von etwa 20 bis 40 °C und einem pH-Wert von etwa 6 bis 9 durchführt, wobei das substrathaltige Medium außerdem bezogen auf das Substrat einen etwa 10- bis 100-fachen molaren Überschuß an Reduktionsäquivalenten enthält.

6. Verfahren nach Anspruch 5, **dadurch gekennzeichnet, dass** man als exogenes Substrat eine Verbindung, ausgewählt unter Indol, n-Hexan, n-Octan, n-Decan, n-Dodecan, Cumol, 1-Methylindol, 5-Cl- oder Br-Indol, Inden, Benzothiophen, alpha-, beta- oder gamma-Ionon, Acridin, Naphthalin, 6-Methyl- oder 8-Methylchinolin, Chinolin und Chinaldin, einsetzt.

7. Verfahren zur mikrobiologischen Produktion von Indigo und/oder Indirubin, **dadurch gekennzeichnet, dass** man
a1) einen rekombinanten, eine Indol-oxidierende Cytochrom P450 Monooxygenase produzierenden Mikroorganismus in einem Kulturmedium, in Gegenwart von exogenem oder intermediär gebildetem Indol, kultiviert; oder
a2) ein Indol-haltiges Reaktionsmedium mit einer Indol-oxidierenden Cytochrom P450 Monooxygenase inkubiert; und
b) das gebildete Oxidationsprodukt oder ein Folgeprodukt davon aus dem Medium isoliert;
wobei die Monooxygenase abgeleitet ist von Cytochrom P450 Monooxygenase BM-3 aus Bacillus megaterium mit einer Aminosäuresequenz gemäß SEQ ID NO:2, welche eine funktionelle Mutation in der Aminosäuresequenzposition 87; oder in den Positionen 87 und 188; oder in den Positionen 87, 188 und 74 aufweist, wobei
Phe87 ersetzt ist durch Ala, Val oder Leu;
Leu188 ersetzt ist durch Asn oder Gln; und
Ala 74 ersetzt ist durch Val oder Gly.

8. Verfahren nach Anspruch 7, **dadurch gekennzeichnet, dass** man aus dem Medium das anfallende Indigo und/oder Indirubin isoliert, welches durch Oxidation von intermediär gebildetem Indol erzeugt wurde.

9. Verfahren nach Anspruch 8, **dadurch gekennzeichnet, dass** man die Indotoxidation durch Kultivierung der Mikroorganismen in Gegenwart von Sauerstoff bei einer Kultivierungstemperatur von etwa 20 bis 40 **°**C und einem pH-Wert von etwa 6 bis 9 durchführt.

10. Verfahren nach einem der vorhergehenden Ansprüche, wobei die Mutante wenigstens eine der folgenden ein- oder mehrfachen Aminosäuresubstitutionen aufweist:
a) Phe87Val;
b) Phe87Val, Leu188Gln; oder
c) Phe87Val, Leu188Gln, Ala74Gly.

11. Verwendung einer Cytochrom P450 Monooxygenase, wobei die Monooxygenase abgeleitet ist von Cytochrom P450 Monooxygenase BM 4 aus Bacillus megaterium mit einer Aminosäuresequenz gemäß SEQ ID NO:2, welche eine funktionelle Mutation in der Aminosäuresequenzposition 87 oder in den Positionen 87 und 188; oder in den Positionen 87, 188 und 74 aufweist, wobei
Phe87 ersetzt ist durch Ala, Val oder Leu;
Leu188 ersetzt ist durch Asn oder Gln; und
Ala 74 ersetzt ist durch Val oder Gly, zur
a) Oxidation gegebenenfalls substituierter N-, O- oder S-heterocyclischen ein- oder mehrkerniger aromatischer Verbindungen;
b) Oxidation gegebenenfalls substituierter ein- oder mehrkerniger Aromaten; ,
c) Oxidation geradkettiger oder verzweigter Alkane und Alkene
d) Oxidation gegebenenfalls substituierter Cycloalkane und Cycloalkene,

12. Verwendung nach Anspruch 11, wobei die Monooxygenase eine der folgen den mehrfachen Aminosäuresubstitutionen aufweist
a) Phe87Val;
b) Phe87Val, Leu188Gln; oder
c) Phe87Val, Leu188Gln, Ala74Gly.

13. Verwendung eines Vektors, umfassend wenigstens ein Expressionskonstrukt enthaltend unter der genetischen-Kontrolle regulativer Nuktleinsäuresequenzen eine kodierende Sequenz, welche eine Nukleinsäuresequenz umfasst, die für eine Monooxygenase nach einem der Ansprüche 11 und 12 kodiert oder eines rekombinanten Mikroorganismus, enthaltend wenigstens einen solchen Vektor zur mikrobiologischen Oxidation von
a) gegebenenfalls substituierten N-, O- oder S-heterocyclischen ein- oder mehrkernigen aromatischen Verbindungen;
b) gegebenenfalls substituierten ein- oder mehrkernigen Aromaten;
c) geradkettigen oder verzweigten Alkanen und Alkenen; und/oder
d) gegebenenfalls substituierten Cycloalkanen und Cycloalkenen.

14. Verwendung nach Anspruch 13, wobei der Mikroorganismus ausgewählt ist unter Bakterien der Gattung Escherichia.

15. Verwendung eines Indol-oxidierenden Cytochrom P450 produzierenden Mikroorganismus gemäß der Definition in Anspruch 13 oder 14 zur Herstellung von Indigo und/oder Indirubin.

16. Bioreaktor, umfassend ein Enzym gemäß der Definition in einem der Ansprüche 11 und 12 oder einen rekombinanten Mikroorganismus gemäß der Definition in einem der Ansprüche 13 und 14 in immobilisierter Form, wobei das verwendete Enzym funktionale Mutationen in den Positionen 87 und 188 oder in den Positionen 87, 188 und 74 aufweist.

## Claims

1. A process for the microbiological oxidation of an N- O- or S-heterocyclic mono- or polynuclear aromatic compound, which comprises
a1) culturing a recombinant cytochrome P450-producing microorganism, transformed by a vector comprising at least one expression construct which carries, under the genetic control of regulatory nucleic acid sequences, a coding sequence for monooxygenase, in a culture medium, in the presence of an exogenous or intermediately formed substrate; or
a2) incubating a substrate-containing reaction medium with a cytochrome P450 monooxygenase of bacterial origin; and
b) isolating the oxidation product formed or a secondary product thereof from the medium;
where the monooxygenase is derived from cytochrome P450 monooxygenase BM-3 from Bacillus megaterium having an amino acid sequence according to SEQ ID NO:2, which has a functional mutation in the amino acid sequence position 87; or in the positions 87 and 188; or in the positions 87, 188 and 74, where
Phe87 is replaced by Ala, Val or Leu;
Leu188 is replaced by Asn or Gln; and
Ala74 is replaced by Val or Gly.

2. The process according to claim 1, wherein the exogenous or intermediately formed substrate is selected from optionally substituted N-, O- or S-heterocyclic mono- or polynuclear aromatic compounds.

3. A process for the microbiological oxidation of a compound selected from optionally substituted mono- or polynuclear aromatics; straight-chain or branched alkanes and alkenes; and optionally substituted cycloalkanes and cycloalkenes;
which comprises
a1) culturing a recombinant cytochrome P450-producing microorganism, transformed by a vector comprising at least one expression construct which carries, under the genetic control of regulatory nucleic acid sequences, a coding sequence for monooxygenase, in a culture medium, in the presence of an exogenous or intermediately formed substrate; or
a2) incubating a substrate-containing reaction medium with a cytochrome P450 monooxygenase; and
b) isolating the oxidation product formed or a secondary product thereof from the medium;
where the monooxygenase is derived from cytochrome P450 monooxygenase BM-3 from Bacillus megaterium having an amino acid sequence according to SEQ ID NO:2, which has a functional mutation in the amino acid sequence position 87; or in the positions 87 and 188; or in the positions 87, 188 and 74, where
Phe87 is replaced by Ala, Val or Leu;
Leu188 is replaced by Asn or Gln; and
Ala74 is replaced by Val or Gly.

4. The process according to claim 3, wherein the exogenous or intermediately formed substrate is selected from:
optionally substituted mono- or polynuclear aromatics;
straight-chain or branched alkanes and alkenes; and
optionally substituted cycloalkanes and cycloalkenes.

5. The process according to any of the preceding claims, wherein, as exogenous substrate, at least one compound selected from the groups of compounds defined above is added to a medium and the oxidation is carried out by enzymatic reaction of the substrate-containing medium in the presence of oxygen at a temperature of approximately 20 to 40°C and a pH of approximately 6 to 9, where the substrate-containing medium additionally comprises an approximately 10- to 100-fold molar excess of reduction equivalents based on the substrate.

6. The process according to claim 5, wherein, as exogenous substrate, a compound selected from indole, n-hexane, n-octane, n-decane, n-dodecane, cumene, 1-methylindole, 5-Cl- or Br-indole, indene, benzothiophene, alpha-, beta- or gamma-ionone, acridine, naphthalene, 6-methyl- or 8-methylquinoline, quinoline and quinaldine is employed.

7. A process for the microbiological production of indigo and/or indirubin, which comprises
a1) culturing a recombinant microorganism which produces an indole-oxidizing cytochrome P450 monooxygenase in a culture medium, in the presence of exogenous or intermediately formed indole; or
a2) incubating an indole-containing reaction medium with an indole-oxidizing cytochrome P450 monooxygenase; and
b) isolating the oxidation product formed or a secondary product thereof from the medium;
where the monooxygenase is derived from cytochrome P450 monooxygenase BM-3 from Bacillus megaterium having an amino acid sequence according to SEQ ID NO:2, which has a functional mutation in the amino acid sequence position 87; or in the positions 87 and 188; or in the positions 87, 188 and 74, where
Phe87 is replaced by Ala, Val or Leu;
Leu188 is replaced by Asn or Gln; and
Ala74 is replaced by Val or Gly.

8. The process according to claim 7, wherein the indigo and/or indirubin obtained, which was produced by oxidation of intermediately formed indole, is isolated from the medium.

9. The process according to claim 8, wherein the indole oxidation is carried out by culturing the microorganisms in the presence of oxygen at a culturing temperature of approximately 20 to 40°C and a pH of approximately 6 to 9.

10. The process according to any of the preceding claims, where the mutant has at least one of the following mono- or polyamino acid substitutions:
a) Phe87Val;
b) Phe87Val, Leu188Gln; or
c) Phe87Val, Leu188Gln, Ala74Gly.

11. The use of a cytochrome P450 monooxygenase, where the monooxygenase is derived from cytochrome P450 monooxygenase BM 4 from Bacillus megaterium having an amino acid sequence according to SEQ ID NO:2, which has a functional mutation in the amino acid sequence position 87 or in the positions 87 and 188; or in the positions 87, 188 and 74, where
Phe87 is replaced by Ala, Val or Leu;
Leu188 is replaced by Asn or Gln; and
Ala74 is replaced by Val or Gly, for
a) oxidation of optionally substituted N-, O- or S-heterocyclic mono- or polynuclear aromatic compounds;
b) oxidation of optionally substituted mono- or polynuclear aromatics;
c) oxidation of straight-chain or branched alkanes and alkenes
d) oxidation of optionally substituted cycloalkanes and cycloalkenes.

12. The use according to claim 11, wherein the monooxy-genase comprises one of the following multiple amino acid substitutions:
a) Phe87Val;
b) Phe87Val, Leu188Gln; or
c) Phe97Val, Leu188Gln, Ala74Gly.

13. The use of a vector comprising at least one expression construct comprising, under the genetic control of regulatory nucleic acid sequences, a coding sequence comprising a nucleic acid sequence coding for a monooxygenase according to either of claims 11 and 12 or of a recombinant microorganism comprising at least one such vector for the microbiological oxidation of
a) optionally substituted N-, 0- or S-heterocyclic mono- or polynuclear aromatic compounds;
b) optionally substituted mono- or polynuclear aromatics;
c) straight-chain or branched alkanes and alkenes; and/or
d) optionally substituted cycloalkanes and cycloalkenes.

14. The use according to claim 13, wherein the microorganism is selected from bacteria of the genus Escherichia.

15. The use of a microorganism producing indole-oxidizing cytochrome P450 according to the definition in claim 13 or 14 for the preparation of indigo and/or indirubin.

16. A bioreactor comprising an enzyme according to the definition in either of claims 11 and 12 or a recombinant microorganism according to the definition in either of claims 13 and 14 in immobilized form, where the enzyme used has functional mutations in the positions 87 and 188 or in the positions 87, 188 and 74.

## Revendications

1. Procédé pour une oxydation microbiologique d'un composé aromatique à un ou plusieurs noyaux, N-hétérocyclique, 0-hétérocyclique ou S-hétérocyclique, **caractérisé en ce qu'**on
a1) cultive un microorganisme recombinant, produisant le Cytochrome P450, transformé avec un vecteur, contenant au moins une construction d'expression qui porte une séquence codant pour une mono-oxygénase sous contrôle génétique de séquences d'acides nucléiques de régulation, dans un milieu de culture, en présence d'un substrat exogène ou formé de manière intermédiaire ; ou
a2) incube un mélange réactionnel contenant un substrat avec une Cytochrome P450 mono-oxygénase d'origine bactérienne ; et
b) isole le produit d'oxydation formé ou un dérivé de celui-ci du milieu ;
la mono-oxygénase étant dérivée de la Cytochrome P450 mono-oxygénase BM-3 de Bacillus megaterium présentant une séquence d'acides aminés selon la SEQ ID NO :2, qui présente une mutation fonctionnelle dans la position d'acide aminé 87 ; ou dans les positions 87 et 188 ; ou dans les positions 87, 188 et 74, où
Phe87 est remplacé par Ala, Val ou Leu ;
Leu188 est remplacé par Asn ou Gln ; et
Ala74 est remplacé par Val ou Gly.

2. Procédé selon la revendication 1, **caractérisé en ce que** le substrat exogène ou formé de manière intermédiaire est choisi parmi les composés aromatiques à un ou plusieurs noyaux, N-hétérocycliques, O-hétérocycliques ou S-hétérocycliques, le cas échéant substitués.

3. Procédé pour l'oxydation microbiologique d'un composé choisi parmi les aromatiques à un ou plusieurs noyaux, le cas échéant substitués ; les alcanes et les alcènes linéaires ou ramifiés ; et les cycloalcanes et les cycloalcènes le cas échéant substitués ;
**caractérisé en ce qu'**on
a1) cultive un microorganisme recombinant, produisant le Cytochrome P450 transformé avec un vecteur, contenant au moins une construction d'expression qui porte une séquence codant pour une mono-oxygénase sous contrôle génétique de séquences d'acides nucléiques de régulation, dans un milieu de culture, en présence d'un substrat exogène ou formé de manière intermédiaire ; ou
a2) incube un mélange réactionnel contenant un substrat avec une Cytochrome P450 mono-oxygénase ; et
b) isole le produit d'oxydation formé ou un dérivé de celui-ci du milieu ;
la mono-oxygénase étant dérivée de la Cytochrome P450 mono-oxygénase BM-3 de Bacillus megaterium présentant une séquence d'acides aminés selon la SEQ ID NO :2, qui présente une mutation fonctionnelle dans la position d'acide aminé 87 ; ou dans les positions 87 et 188 ; ou dans les positions 87, 188 et 74, où
Phe87 est remplacé par Ala, Val ou Leu ;
Leu188 est remplacé par Asn ou Gln ; et
Ala74 est remplacé par Val ou Gly.

4. Procédé selon la revendication 3, **caractérisé en ce que** le substrat exogène ou formé de manière intermédiaire est choisi parmi :
- les aromatiques à un ou plusieurs noyaux le cas échéant substitués ; les alcanes et les alcènes linéaires ou ramifiés ; et
- les cycloalcanes et les cycloalcènes le cas échéant substitués.

5. Procédé selon l'une quelconque des revendications précédentes, **caractérisé en ce qu'**on ajoute, comme substrat exogène, au moins un composé, choisi parmi les groupes définis ci-dessus de composés, à un milieu et on réalise l'oxydation par transformation enzymatique du milieu contenant le substrat en présence d'oxygène à une température d'environ 20 à 40°C et à un pH d'environ 6 à 9, le milieu contenant le substrat contenant en outre, par rapport au substrat, un excès molaire, d'un facteur d'environ 10 à 100, d'équivalents de réduction.

6. Procédé selon la revendication 5, **caractérisé en ce qu'**on utilise comme substrat exogène un composé, choisi parmi l'indole, le n-hexane, le n-octane, le n-décane, le n-dodécane, le cumène, le 1-méthylindole, le 5-Cl-indole ou le Br-indole, l'indène, le benzothiophène, l'alpha-ionone, la bêta-ionone ou la gamma-ionone, l'acridine, le naphtalène, la 6-méthylquinoléine ou la 8-méthylquinoléine, la quinoléine et la quinaldine.

7. Procédé pour la production microbiologique d'indigo et/ou d'indirubine, **caractérisé en ce qu'**on
a1) cultive un microorganisme recombinant, produisant une Cytochrome P450 mono-oxygénase oxydant l'indole dans un milieu de culture, en présence d'indole exogène ou formé de manière intermédiaire ; ou
a2) incube un mélange réactionnel contenant de l'indole avec une Cytochrome P450 mono-oxygénase oxydant l'indole ; et
b) isole le produit d'oxydation formé ou un dérivé de celui-ci du milieu ;
la mono-oxygénase étant dérivée de la Cytochrome P450 mono-oxygénase BM-3 de Bacillus megaterium présentant une séquence d'acides aminés selon la SEQ ID NO :2, qui présente une mutation fonctionnelle dans la position d'acide aminé 87 ; ou dans les positions 87 et 188 ; ou dans les positions 87, 188 et 74, où
Phe87 est remplacé par Ala, Val ou Leu ;
Leu188 est remplacé par Asn ou Gln ; et
Ala74 est remplacé par Val ou Gly.

8. Procédé selon la revendication 7, **caractérisé en ce qu'**on isole à partir du milieu l'indigo et/ou l'indirubine produit(e) qui a été formé(e) par oxydation de l'indole formé de manière intermédiaire.

9. Procédé selon la revendication 8, **caractérisé en ce qu'**on réalise l'oxydation de l'indole par culture des microorganismes en présence d'oxygène à une température de culture d'environ 20 à 40°C et à un pH d'environ 6 à 9.

10. Procédé selon l'une quelconque des revendications précédentes, le mutant présentant au moins une des substitutions simples ou multiples d'acides aminés suivantes :
a) Phe87Val ;
b) Phe87Val, Leu188Gln ; ou
c) Phe87Val, Leu188Gln, Ala74Gly.

11. Utilisation d'une Cytochrome P450 mono-oxygénase, la mono-oxygénase étant dérivée de la Cytochrome P450 mono-oxygénase BM-4 de Bacillus megaterium présentant une séquence d'acides aminés selon la SEQ ID NO :2, qui présente une mutation fonctionnelle dans la position d'acide aminé 87 ; ou dans les positions 87 et 188 ; ou dans les positions 87, 188 et 74, où
Phe87 est remplacé par Ala, Val ou Leu ;
Leu188 est remplacé par Asn ou Gln ; et
Ala74 est remplacé par Val ou Gly, en vue de
a) l'oxydation de composés aromatiques à un ou plusieurs noyaux, N-hétérocycliques, O-hétérocycliques ou S-hétérocycliques, le cas échéant substitués ;
b) l'oxydation de composés aromatiques à un ou plusieurs noyaux, le cas échéant substitués ;
c) l'oxydation d'alcanes et d'alcènes linéaires ou ramifiés ;
d) l'oxydation de cycloalcanes et de cycloalcènes le cas échéant substitués.

12. Utilisation selon la revendication 11, où la mono-oxygénase présente une des substitutions multiples suivantes
a) Phe87Val ;
b) Phe87Val, Leu188Gln ; ou
c) Phe87Val, Leu188Gln, Ala74Gly.

13. Utilisation d'un vecteur, comprenant au moins une construction d'expression contenant une séquence codante sous contrôle génétique de séquences d'acides nucléiques de régulation, qui comprend une séquence d'acides nucléiques qui code pour une mono-oxygénase selon l'une quelconque des revendications 11 et 12 ou d'un microorganisme recombinant contenant au moins un tel vecteur pour l'oxydation microbiologique
a) de composés aromatiques à un ou plusieurs noyaux, N-hétérocycliques, 0-hétérocycliques ou S-hétérocycliques, le cas échéant substitués ;
b) de composés aromatiques à un ou plusieurs noyaux, le cas échéant substitués ;
c) d'alcanes ou d'alcènes linéaires ou ramifiés ; et/ou
d) de cycloalcanes et de cycloalcènes le cas échéant substitués.

14. Utilisation selon la revendication 13, le microorganisme étant choisi parmi les bactéries du genre Escherichia.

15. Utilisation d'un microorganisme produisant du Cytochrome P450 oxydant l'indole selon la définition dans la revendication 13 ou 14 pour la préparation d'indigo et/ou d'indirubine.

16. Bioréacteur, comprenant une enzyme selon la définition dans l'une quelconque des revendications 11 et 12 ou un microorganisme recombinant selon la définition dans l'une quelconque des revendications 13 et 14 dans une forme immobilisée, l'enzyme utilisée présentant des mutations fonctionnelles dans les positions 87 et 188 ou dans les positions 87, 188 et 74.


### SEQUENZPROTOKOLL

<110> BASF Aktiengesellschaft
<120> Neue Cytochrom P450 Monooxygenasen und deren Verwendung zur Oxidation von organischen Substraten
<130> M/40241
<140>
   <141>
<160> 9
<170> PatentIn Ver. 2.1
<210> 1
   <211> 3150
   <212> DNA
   <213> Bacillus megaterium
<220>
   <221> CDS
   <222> (4)..(3150)
<400> 1
   atg aca att aaa gaa atg cct cag cca aaa acg ttt gga gag ctt aaa     48
       Thr Ile Lys Glu Met Pro Gln Pro Lys Thr Phe Gly Glu Leu Lys
         1               5                  10                  15
   aat tta ccg tta tta aac aca gat aaa ccg gtt caa gct ttg atg aaa     96
   Asn Leu Pro Leu Leu Asn Thr Asp Lys Pro Val Gln Ala Leu Met Lys
                       20                  25                  30
   att gcg gat gaa tta gga gaa atc ttt aaa ttc gag gcg cct ggt cgt    144
   Ile Ala Asp Glu Leu Gly Glu Ile Phe Lys Phe Glu Ala Pro Gly Arg
                   35                  40                  45
   gta acg cgc tac tta tca agt cag cgt cta att aaa gaa gca tgc gat    192
   Val Thr Arg Tyr Leu Ser Ser Gln Arg Leu Ile Lys Glu Ala Cys Asp
               50                  55                  60
   gaa tca cgc ttt gat aaa aac tta agt caa gcg ctt aaa ttt gta cgt    240
   Glu Ser Arg Phe Asp Lys Asn Leu Ser Gln Ala Leu Lys Phe Val Arg
           65                  70                  75
   gat ttt gca gga gac ggg tta ttt aca agc tgg acg cat gaa aaa aat    288
   Asp Phe Ala Gly Asp Gly Leu Phe Thr Ser Trp Thr His Glu Lys Asn
    80                  85                  90                  95
   tgg aaa aaa gcg cat aat atc tta ctt cca agc ttc agt cag cag gca    336
   Trp Lys Lys Ala His Asn Ile Leu Leu Pro Ser Phe Ser Gln Gln Ala
                   100                 105                 110
   atg aaa ggc tat cat gcg atg atg gtc gat atc gcc gtg cag ctt gtt    384
   Met Lys Gly Tyr His Ala Met Met Val Asp Ile Ala Val Gln Leu Val
               115                 120                 125
   caa aag tgg gag cgt cta aat gca gat gag cat att gaa gta ccg gaa    432
   Gln Lys Trp Glu Arg Leu Asn Ala Asp Glu His Ile Glu Val Pro Glu
           130                 135                 140
   gac atg aca cgt tta acg ctt gat aca att ggt ctt tgc ggc ttt aac    480
   Asp Met Thr Arg Leu Thr Leu Asp Thr Ile Gly Leu Cys Gly Phe Asn
       145                 150                 155
   tat cgc ttt aac agc ttt tac cga gat cag cct cat cca ttt att aca    528
   Tyr Arg Phe Asn Ser Phe Tyr Arg Asp Gln Pro His Pro Phe Ile Thr
   160                 165                 170                 175
   agt atg gtc cgt gca ctg gat gaa gca atg aac aag ctg cag cga gca    576
   Ser Met Val Arg Ala Leu Asp Glu Ala Met Asn Lys Leu Gln Arg Ala
                   180                 185                 190
   aat cca gac gac cca gct tat gat gaa aac aag cgc cag ttt caa gaa    624
   Asn Pro Asp Asp Pro Ala Tyr Asp Glu Asn Lys Arg Gln Phe Gln Glu
               195                 200                 205
   gat atc aag gtg atg aac gac cta gta gat aaa att att gca gat cgc    672
   Asp Ile Lys Val Met Asn Asp Leu Val Asp Lys Ile Ile Ala Asp Arg
           210                 215                 220
   aaa gca agc ggt gaa caa agc gat gat tta tta acg cat atg cta aac    720
   Lys Ala Ser Gly Glu Gln Ser Asp Asp Leu Leu Thr His Met Leu Asn
       225                 230                 235
   gga aaa gat cca gaa acg ggt gag ccg ctt gat gac gag aac att cgc    768
   Gly Lys Asp Pro Glu Thr Gly Glu Pro Leu Asp Asp Glu Asn Ile Arg
   240                 245                 250                 255
   tat caa att att aca ttc tta att gcg gga cac gaa aca aca agt ggt    816
   Tyr Gln Ile Ile Thr Phe Leu Ile Ala Gly His Glu Thr Thr Ser Gly
                   260                 265                 270
   ctt tta tca ttt gcg ctg tat ttc tta gtg aaa aat cca cat gta tta    864
   Leu Leu Ser Phe Ala Leu Tyr Phe Leu Val Lys Asn Pro His Val Leu
               275                 280                 285
   caa aaa gca gca gaa gaa gca gca cga gtt cta gta gat cct gtt cca    912
   Gln Lys Ala Ala Glu Glu Ala Ala Arg Val Leu Val Asp Pro Val Pro
           290                 295                 300
   agc tac aaa caa gtc aaa cag ctt aaa tat gtc ggc atg gtc tta aac     960
   Ser Tyr Lys Gln Val Lys Gln Leu Lys Tyr Val Gly Met Val Leu Asn
       305                 310                 315
   gaa gcg ctg cgc tta tgg cca act gct cct gcg ttt tcc cta tat gca    1008
   Glu Ala Leu Arg Leu Trp Pro Thr Ala Pro Ala Phe Ser Leu Tyr Ala
   320                 325                 330                 335
   aaa gaa gat acg gtg ctt gga gga gaa tat cct tta gaa aaa ggc gac    1056
   Lys Glu Asp Thr Val Leu Gly Gly Glu Tyr Pro Leu Glu Lys Gly Asp
                   340                 345                 350
   gaa cta atg gtt ctg att cct cag ctt cac cgt gat aaa aca att tgg    1104
   Glu Leu Met Val Leu Ile Pro Gln Leu His Arg Asp Lys Thr Ile Trp
               355                 360                 365
   gga gac gat gtg gaa gag ttc cgt cca gag cgt ttt gaa aat cca agt    1152
   Gly Asp Asp Val Glu Glu Phe Arg Pro Glu Arg Phe Glu Asn Pro Ser
           370                 375                 380
   gcg att ccg cag cat gcg ttt aaa ccg ttt gga aac ggt cag cgt gcg    1200
   Ala Ile Pro Gln His Ala Phe Lys Pro Phe Gly Asn Gly Gln Arg Ala
       385                 390                 395
   tgt atc ggt cag cag ttc gct ctt cat gaa gca acg ctg gta ctt ggt    1248
   Cys Ile Gly Gln Gln Phe Ala Leu His Glu Ala Thr Leu Val Leu Gly
   400                 405                 410                 415
   atg atg cta aaa cac ttt gac ttt gaa gat cat aca aac tac gag ctg    1296
   Met Met Leu Lys His Phe Asp Phe Glu Asp His Thr Asn Tyr Glu Leu
                   420                 425                 430
   gat att aaa gaa act tta acg tta aaa cct gaa ggc ttt gtg gta aaa    1344
   Asp Ile Lys Glu Thr Leu Thr Leu Lys Pro Glu Gly Phe Val Val Lys
               435                 440                 445
   gca aaa tcg aaa aaa att ccg ctt ggc ggt att cct tca cct agc act    1392
   Ala Lys Ser Lys Lys Ile Pro Leu Gly Gly Ile Pro Ser Pro Ser Thr
           450                 455                 460
   gaa cag tct gct aaa aaa gta cgc aaa aag gca gaa aac gct cat aat    1440
   Glu Gln Ser Ala Lys Lys Val Arg Lys Lys Ala Glu Asn Ala His Asn
       465                 470                 475
   acg ccg ctg ctt gtg cta tac ggt tca aat atg gga aca gct gaa gga    1488
   Thr Pro Leu Leu Val Leu Tyr Gly Ser Asn Met Gly Thr Ala Glu Gly
   480                 485                 490                 495
   acg gcg cgt gat tta gca gat att gca atg agc aaa gga ttt gca ccg    1536
   Thr Ala Arg Asp Leu Ala Asp Ile Ala Met Ser Lys Gly Phe Ala Pro
                   500                 505                 510
   cag gtc gca acg ctt gat tca cac gcc gga aat ctt ccg cgc gaa gga      1584
   Gln Val Ala Thr Leu Asp Ser His Ala Gly Asn Leu Pro Arg Glu Gly
               515                 520                 525
   gct gta tta att gta acg gcg tct tat aac ggt cat ccg cct gat aac      1632
   Ala Val Leu Ile Val Thr Ala Ser Tyr Asn Gly His Pro Pro Asp Asn
           530                 535                 540
   gca aag caa ttt gtc gac tgg tta gac caa gcg tct gct gat gaa gta      1680
   Ala Lys Gln Phe Val Asp Trp Leu Asp Gln Ala Ser Ala Asp Glu Val
       545                 550                 555
   aaa ggc gtt cgc tac tcc gta ttt gga tgc ggc gat aaa aac tgg gct      1728
   Lys Gly Val Arg Tyr Ser Val Phe Gly Cys Gly Asp Lys Asn Trp Ala
   560                 565                 570                 575
   act acg tat caa aaa gtg cct gct ttt atc gat gaa acg ctt gcc gct      1776
   Thr Thr Tyr Gln Lys Val Pro Ala Phe Ile Asp Glu Thr Leu Ala Ala
                   580                 585                 590
   aaa ggg gca gaa aac atc gct gac cgc ggt gaa gca gat gca agc gac      1824
   Lys Gly Ala Glu Asn Ile Ala Asp Arg Gly Glu Ala Asp Ala Ser Asp
               595                 600                 605
   gac ttt gaa ggc aca tat gaa gaa tgg cgt gaa cat atg tgg agt gac      1872
   Asp Phe Glu Gly Thr Tyr Glu Glu Trp Arg Glu His Met Trp Ser Asp
           610                 615                 620
   gta gca gcc tac ttt aac ctc gac att gaa aac agt gaa gat aat aaa      1920
   Val Ala Ala Tyr Phe Asn Leu Asp Ile Glu Asn Ser Glu Asp Asn Lys
       625                 630                 635
   tct act ctt tca ctt caa ttt gtc gac agc gcc gcg gat atg ccg ctt      1968
   Ser Thr Leu Ser Leu Gln Phe Val Asp Ser Ala Ala Asp Met Pro Leu
   640                 645                 650                 655
   gcg aaa atg cac ggt gcg ttt tca acg aac gtc gta gca agc aaa gaa      2016
   Ala Lys Met His Gly Ala Phe Ser Thr Asn Val Val Ala Ser Lys Glu
                   660                 665                 670
   ctt caa cag cca ggc agt gca cga agc acg cga cat ctt gaa att gaa      2064
   Leu Gln Gln Pro Gly Ser Ala Arg Ser Thr Arg His Leu Glu Ile Glu
               675                 680                 685
   ctt cca aaa gaa gct tct tat caa gaa gga gat cat tta ggt gtt att      2112
   Leu Pro Lys Glu Ala Ser Tyr Gln Glu Gly Asp His Leu Gly Val Ile
           690                 695                 700
   cct cgc aac tat gaa gga ata gta aac cgt gta aca gca agg ttc ggc      2160
   Pro Arg Asn Tyr Glu Gly Ile Val Asn Arg Val Thr Ala Arg Phe Gly
       705                 710                 715
   cta gat gca tca cag caa atc cgt ctg gaa gca gaa gaa gaa aaa tta     2208
   Leu Asp Ala Ser Gln Gln Ile Arg Leu Glu Ala Glu Glu Glu Lys Leu
   720                 725                 730                 735
   gct cat ttg cca ctc gct aaa aca gta tcc gta gaa gag ctt ctg caa     2256
   Ala His Leu Pro Leu Ala Lys Thr Val Ser Val Glu Glu Leu Leu Gln
                   740                 745                 750
   tac gtg gag ctt caa gat cct gtt acg cgc acg cag ctt cgc gca atg     2304
   Tyr Val Glu Leu Gln Asp Pro Val Thr Arg Thr Gln Leu Arg Ala Met
               755                 760                 765
   gct gct aaa acg gtc tgc ccg ccg cat aaa gta gag ctt gaa gcc ttg     2352
   Ala Ala Lys Thr Val Cys Pro Pro His Lys Val Glu Leu Glu Ala Leu
           770                 775                 780
   ctt gaa aag caa gcc tac aaa gaa caa gtg ctg gca aaa cgt tta aca     2400
   Leu Glu Lys Gln Ala Tyr Lys Glu Gln Val Leu Ala Lys Arg Leu Thr
       785                 790                 795
   atg ctt gaa ctg ctt gaa aaa tac ccg gcg tgt gaa atg aaa ttc agc     2448
   Met Leu Glu Leu Leu Glu Lys Tyr Pro Ala Cys Glu Met Lys Phe Ser
   800                 805                 810                 815
   gaa ttt atc gcc ctt ctg cca agc ata cgc ccg cgc tat tac tcg att     2496
   Glu Phe Ile Ala Leu Leu Pro Ser Ile Arg Pro Arg Tyr Tyr Ser Ile
                   820                 825                 830
   tct tca tca cct cgt gtc gat gaa aaa caa gca agc atc acg gtc agc     2544
   Ser Ser Ser Pro Arg Val Asp Glu Lys Gln Ala Ser Ile Thr Val Ser
               835                 840                 845
   gtt gtc tca gga gaa gcg tgg agc gga tat gga gaa tat aaa gga att     2592
   Val Val Ser Gly Glu Ala Trp Ser Gly Tyr Gly Glu Tyr Lys Gly Ile
           850                 855                 860
   gcg tcg aac tat ctt gcc gag ctg caa gaa gga gat acg att acg tgc     2640
   Ala Ser Asn Tyr Leu Ala Glu Leu Gln Glu Gly Asp Thr Ile Thr Cys
       865                 870                 875
   ttt att tcc aca ccg cag tca gaa ttt acg ctg cca aaa gac cct gaa     2688
   Phe Ile Ser Thr Pro Gln Ser Glu Phe Thr Leu Pro Lys Asp Pro Glu
   880                 885                 890                 895
   acg ccg ctt atc atg gtc gga ccg gga aca ggc gtc gcg ccg ttt aga     2736
   Thr Pro Leu Ile Met Val Gly Pro Gly Thr Gly Val Ala Pro Phe Arg
                   900                 905                 910
   ggc ttt gtg cag gcg cgc aaa cag cta aaa gaa caa gga cag tca ctt     2784
   Gly Phe Val Gln Ala Arg Lys Gln Leu Lys Glu Gln Gly Gln Ser Leu
               915                 920                 925
   gga gaa gca cat tta tac ttc ggc tgc cgt tca cct cat gaa gac tat     2832
   Gly Glu Ala His Leu Tyr Phe Gly Cys Arg Ser Pro His Glu Asp Tyr
           930                 935                 940
   ctg tat caa gaa gag ctt gaa aac gcc caa agc gaa ggc atc att acg     2880
   Leu Tyr Gln Glu Glu Leu Glu Asn Ala Gln Ser Glu Gly Ile Ile Thr
       945                 950                 955
   ctt cat acc gct ttt tct cgc atg cca aat cag ccg aaa aca tac gtt     2928
   Leu His Thr Ala Phe Ser Arg Met Pro Asn Gln Pro Lys Thr Tyr Val
   960                 965                 970                 975
   cag cac gta atg gaa caa gac ggc aag aaa ttg att gaa ctt ctt gat     2976
   Gln His Val Met Glu Gln Asp Gly Lys Lys Leu Ile Glu Leu Leu Asp
                   980                 985                 990
   caa gga gcg cac ttc tat att tgc gga gac gga agc caa atg gca cct     3024
   Gln Gly Ala His Phe Tyr Ile Cys Gly Asp Gly Ser Gln Met Ala Pro
               995                 1000                1005
   gcc gtt gaa gca acg ctt atg aaa agc tat gct gac gtt cac caa gtg     3072
   Ala Val Glu Ala Thr Leu Met Lys Ser Tyr Ala Asp Val His Gln Val
           1010                1015                1020
   agt gaa gca gac gct cgc tta tgg ctg cag cag cta gaa gaa aaa ggc     3120
   Ser Glu Ala Asp Ala Arg Leu Trp Leu Gln Gln Leu Glu Glu Lys Gly
       1025                1030                1035
   cga tac gca aaa gac gtg tgg gct ggg taa                             3150
   Arg Tyr Ala Lys Asp Val Trp Ala Gly
   1040                1045
<210> 2
   <211> 1048
   <212> PRT
   <213> Bacillus megaterium
<400> 2
   Thr Ile Lys Glu Met Pro Gln Pro Lys Thr Phe Gly Glu Leu Lys Asn
   1               5                   10                  15
   Leu Pro Leu Leu Asn Thr Asp Lys Pro Val Gln Ala Leu Met Lys Ile
               20                  25                  30
   Ala Asp Glu Leu Gly Glu Ile Phe Lys Phe Glu Ala Pro Gly Arg Val
           35                  40                  45
   Thr Arg Tyr Leu Ser Ser Gln Arg Leu Ile Lys Glu Ala Cys Asp Glu
       50                  55                  60
   Ser Arg Phe Asp Lys Asn Leu Ser Gln Ala Leu Lys Phe Val Arg Asp
   65 70 75 80
   Phe Ala Gly Asp Gly Leu Phe Thr Ser Trp Thr His Glu Lys Asn Trp
   85 90 95
   Lys Lys Ala His Asn Ile Leu Leu Pro Ser Phe Ser Gln Gln Ala Met
   100 105 110
   Lys Gly Tyr His Ala Met Met Val Asp Ile Ala Val Gln Leu Val Gln
   115 120 125
   Lys Trp Glu Arg Leu Asn Ala Asp Glu His Ile Glu Val Pro Glu Asp
   130 135 140
   Met Thr Arg Leu Thr Leu Asp Thr Ile Gly Leu Cys Gly Phe Asn Tyr
   145 150 155 160
   Arg Phe Asn Ser Phe Tyr Arg Asp Gln Pro His Pro Phe Ile Thr Ser
   165 170 175
   Met Val Arg Ala Leu Asp Glu Ala Met Asn Lys Leu Gln Arg Ala Asn
   180 185 190
   Pro Asp Asp Pro Ala Tyr Asp Glu Asn Lys Arg Gln Phe Gln Glu Asp
   195 200 205
   Ile Lys Val Met Asn Asp Leu Val Asp Lys Ile Ile Ala Asp Arg Lys
   210 215 220
   Ala Ser Gly Glu Gln Ser Asp Asp Leu Leu Thr His Met Leu Asn Gly
   225 230 235 240
   Lys Asp Pro Glu Thr Gly Glu Pro Leu Asp Asp Glu Asn Ile Arg Tyr
   245 250 255
   Gln Ile Ile Thr Phe Leu Ile Ala Gly His Glu Thr Thr Ser Gly Leu
   260 265 270
   Leu Ser Phe Ala Leu Tyr Phe Leu Val Lys Asn Pro His Val Leu Gln
   275 280 285
   Lys Ala Ala Glu Glu Ala Ala Arg Val Leu Val Asp Pro Val Pro Ser
   290 295 300
   Tyr Lys Gln Val Lys Gln Leu Lys Tyr Val Gly Met Val Leu Asn Glu
   305 310 315 320
   Ala Leu Arg Leu Trp Pro Thr Ala Pro Ala Phe Ser Leu Tyr Ala Lys
   325 330 335
   Glu Asp Thr Val Leu Gly Gly Glu Tyr Pro Leu Glu Lys Gly Asp Glu
   340 345 350
   Leu Met Val Leu Ile Pro Gln Leu His Arg Asp Lys Thr Ile Trp Gly
   355 360 365
   Asp Asp Val Glu Glu Phe Arg Pro Glu Arg Phe Glu Asn Pro Ser Ala
   370 375 380
   Ile Pro Gln His Ala Phe Lys Pro Phe Gly Asn Gly Gln Arg Ala Cys
   385 390 395 400
   Ile Gly Gln Gln Phe Ala Leu His Glu Ala Thr Leu Val Leu Gly Met
   405 410 415
   Met Leu Lys His Phe Asp Phe Glu Asp His Thr Asn Tyr Glu Leu Asp
   420 425 430
   Ile Lys Glu Thr Leu Thr Leu Lys Pro Glu Gly Phe Val Val Lys Ala
   435 440 445
   Lys Ser Lys Lys Ile Pro Leu Gly Gly Ile Pro Ser Pro Ser Thr Glu
   450 455 460
   Gln Ser Ala Lys Lys Val Arg Lys Lys Ala Glu Asn Ala His Asn Thr
   465 470 475 480
   Pro Leu Leu Val Leu Tyr Gly Ser Asn Met Gly Thr Ala Glu Gly Thr
   485 490 495
   Ala Arg Asp Leu Ala Asp Ile Ala Met Ser Lys Gly Phe Ala Pro Gln
   500 505 510
   Val Ala Thr Leu Asp Ser His Ala Gly Asn Leu Pro Arg Glu Gly Ala
   515 520 525
   Val Leu Ile Val Thr Ala Ser Tyr Asn Gly His Pro Pro Asp Asn Ala
   530 535 540
   Lys Gln Phe Val Asp Trp Leu Asp Gln Ala Ser Ala Asp Glu Val Lys
   545 550 555 560
   Gly Val Arg Tyr Ser Val Phe Gly Cys Gly Asp Lys Asn Trp Ala Thr
   565 570 575
   Thr Tyr Gln Lys Val Pro Ala Phe Ile Asp Glu Thr Leu Ala Ala Lys
   580 585 590
   Gly Ala Glu Asn Ile Ala Asp Arg Gly Glu Ala Asp Ala Ser Asp Asp
   595 600 605
   Phe Glu Gly Thr Tyr Glu Glu Trp Arg Glu His Met Trp Ser Asp Val
   610 615 620
   Ala Ala Tyr Phe Asn Leu Asp Ile Glu Asn Ser Glu Asp Asn Lys Ser
   625 630 635 640
   Thr Leu Ser Leu Gln Phe Val Asp Ser Ala Ala Asp Met Pro Leu Ala
   645 650 655
   Lys Met His Gly Ala Phe Ser Thr Asn Val Val Ala Ser Lys Glu Leu
   660 665 670
   Gln Gln Pro Gly Ser Ala Arg Ser Thr Arg His Leu Glu Ile Glu Leu
   675 680 685
   Pro Lys Glu Ala Ser Tyr Gln Glu Gly Asp His Leu Gly Val Ile Pro
   690 695 700
   Arg Asn Tyr Glu Gly Ile Val Asn Arg Val Thr Ala Arg Phe Gly Leu
   705 710 715 720
   Asp Ala Ser Gln Gln Ile Arg Leu Glu Ala Glu Glu Glu Lys Leu Ala
   725 730 735
   His Leu Pro Leu Ala Lys Thr Val Ser Val Glu Glu Leu Leu Gln Tyr
   740 745 750
   Val Glu Leu Gln Asp Pro Val Thr Arg Thr Gln Leu Arg Ala Met Ala
   755 760 765
   Ala Lys Thr Val Cys Pro Pro His Lys Val Glu Leu Glu Ala Leu Leu
   770 775 780
   Glu Lys Gln Ala Tyr Lys Glu Gln Val Leu Ala Lys Arg Leu Thr Met
   785 790 795 800
   Leu Glu Leu Leu Glu Lys Tyr Pro Ala Cys Glu Met Lys Phe Ser Glu
   805 810 815
   Phe Ile Ala Leu Leu Pro Ser Ile Arg Pro Arg Tyr Tyr Ser Ile Ser
   820 825 830
   Ser Ser Pro Arg Val Asp Glu Lys Gln Ala Ser Ile Thr Val Ser Val
   835 840 845
   Val Ser Gly Glu Ala Trp Ser Gly Tyr Gly Glu Tyr Lys Gly Ile Ala
   850 855 860
   Ser Asn Tyr Leu Ala Glu Leu Gln Glu Gly Asp Thr Ile Thr Cys Phe
   865 870 875 880
   Ile Ser Thr Pro Gln Ser Glu Phe Thr Leu Pro Lys Asp Pro Glu Thr
                   885                 890                 895
   Pro Leu Ile Met Val Gly Pro Gly Thr Gly Val Ala Pro Phe Arg Gly
               900                 905                 910
   Phe Val Gln Ala Arg Lys Gln Leu Lys Glu Gln Gly Gln Ser Leu Gly
           915                 920                 925
   Glu Ala His Leu Tyr Phe Gly Cys Arg Ser Pro His Glu Asp Tyr Leu
       930                 935                 940
   Tyr Gln Glu Glu Leu Glu Asn Ala Gln Ser Glu Gly Ile Ile Thr Leu
   945                 950                 955                 960
   His Thr Ala Phe Ser Arg Met Pro Asn Gln Pro Lys Thr Tyr Val Gln
                   965                 970                 975
   His Val Met Glu Gln Asp Gly Lys Lys Leu Ile Glu Leu Leu Asp Gln
               980                 985                 990
   Gly Ala His Phe Tyr Ile Cys Gly Asp Gly Ser Gln Met Ala Pro Ala
           995                 1000                1005
   Val Glu Ala Thr Leu Met Lys Ser Tyr Ala Asp Val His Gln Val Ser
       1010                1015                1020
   Glu Ala Asp Ala Arg Leu Trp Leu Gln Gln Leu Glu Glu Lys Gly Arg
   025                 1030                1035                1040
   Tyr Ala Lys Asp Val Trp Ala Gly
                   1045
<210> 3
   <211> 30
   <212> DNA
   <213> Synthetic sequence
<220>
   <223> Description of the synthetic sequence: PCR primer
<400> 3
   gcaggagacg ggttgnnnac aagctggacg 30
<210> 4
   <211> 30
   <212> DNA
   <213> Synthetic sequence
<220>
   <223> Description of the synthetic sequence: PCR primer
<400> 4
   cgtccagctt gtnnncaacc cgtctcctgc 30
<210> 5
   <211> 34
   <212> DNA
   <213> Synthetic sequence
<220>
   <223> Description of the synthetic sequence: PCR primer
<400> 5
   gaagcaatga acaagnnnca gcgagcaaat ccag 34
<210> 6
   <211> 30
   <212> DNA
   <213> Synthetic sequence
<220>
   <223> Description of the synthetic sequence: PCR primer
<400> 6
   ctggatttgc tcgctgnnnc ttgttcattg 30
<210> 7
   <211> 41
   <212> DNA
   <213> Synthetic sequence
<220>
   <223> Description of the synthetic sequence: PCR primer
<400> 7
   gctttgataa aaacttaaag tcaannnctt aaatttgtac g 41
<210> 8
   <211> 40
   <212> DNA
   <213> Synthetic sequence
<220>
   <223> Description of the synthetic sequence: PCR primer
<400> 8
   cgtacaaatt taagnnnttg acttaagttt ttatcaaagc 40
<210> 9
   <211> 1049
   <212> PRT
   <213> Bacillus megaterium
<400> 9